(19) Europäisches Patentamt
European Patent Office
Office européen des brevets

(11) EP 1 727 865 B1

(12) EUROPÄISCHE PATENTSCHRIFT

(45) Veröffentlichungstag und Bekanntmachung des Hinweises auf die Patenterteilung:
**19.05.2010 Patentblatt 2010/20**

(21) Anmeldenummer: **05716184.6**

(22) Anmeldetag: **17.03.2005**

(51) Int Cl.:
***C09C 1/30*** *(2006.01)* ***A61K 9/107*** *(2006.01)*

(86) Internationale Anmeldenummer:
**PCT/EP2005/002887**

(87) Internationale Veröffentlichungsnummer:
**WO 2005/092989 (06.10.2005 Gazette 2005/40)**

# (54) PARTIKELSTABILISIERTE EMULSIONEN

PARTICLE-STABILISED EMULSIONS

EMULSIONS A PARTICULES STABILISEES

(84) Benannte Vertragsstaaten:
**BE DE FR GB**

(30) Priorität: **25.03.2004 DE 102004014704**

(43) Veröffentlichungstag der Anmeldung:
**06.12.2006 Patentblatt 2006/49**

(73) Patentinhaber: **Wacker Chemie AG**
**81737 München (DE)**

(72) Erfinder:
- **GOTTSCHALK-GAUDIG, Torsten**
**84561 Mehring (DE)**
- **BARTHEL, Herbert**
**84547 Emmerting (DE)**
- **BINKS, Bernard Paul**
**Walkington Yorkshire HU17 8XX (GB)**
- **HOROZOV, Tommy, S.**
**Beverley Yorkshire HU17 0AZ (GB)**

(74) Vertreter: **Gössmann, Christoph Tassilo et al**
**Wacker-Chemie GmbH**
**Zentralbereich PML,**
**Hanns-Seidel-Platz 4**
**81737 München (DE)**

(56) Entgegenhaltungen:
**EP-A- 1 433 749** **DE-A1- 10 238 649**
**US-A1- 2003 175 317**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Emulsionen vom Typ Wasser-in-Öl (W/O) bzw. Öl-in-Wasser (O/W)und deren Herstellung.

Emulsionen, entweder als Wasser-in-Öl- (W/O) oder Öl-in-Wasser-(O/W) Dispersion finden weite Verbreitung als Applikationsform für Beschichtungsstoffe wie z.B. wasserbasierende Farben und Lacke, als Kleb- und Dichtstoffe wie z.B. wäßrige Epoxy- oder Polyurethansysteme, als kosmetische Formulierungen, als Reinigungs- und Desinfektionsmittel, in der Nahrungsmittelindustrie, zur Oberflächenmodifizierung fester oder flüssiger Substrate oder als Reaktionsmedien bei der Emulsionspolymerisation.

Im allgemeinen erfolgt die Dispergierung und Stabilisierung der dispersen Phase mit Hilfe von Emulgatoren. Es werden kationische, anionische, ampholytische sowie nichtionische Emulgatoren verwendet. Den Emulgatoren ist gemeinsam, dass sie grenzflächenaktive Stoffe darstellen. D.h. sie lagern sich bevorzugt an Grenzflächen wie z.B. flüssig-flüssig, flüssigfest oder flüssig-gasförmig an und verringern dadurch die Grenzflächen- / Oberflächenenergie. Bei der Applikation der Emulsion können die Emulgatoren jedoch auch die Oberfläche des zu behandelnden Substrats bedecken und dabei die Benetzungseigenschaften der Oberfläche stark verändern. Dies kann z.B. die Adhäsionseigenschaften eines Beschichtungsstoffs oder einer Kleb- oder Dichtfuge negativ beeinflussen. Ferner kann die Wiederüberstreichbarkeit negativ beeinflusst werden. Zudem stellen Emulgatoren auf der Basis von organischen Molekülen potentielle Gefahrstoffe beim Einsatz in pharmazeutischen oder kosmetischen Formulierungen oder in Nahrungsmitteln dar.

Pickering beschrieb 1907 erstmalig die Herstellung von Emulsionen, die nur durch Zusatz verschiedener Feststoffe, wie basischer Kupfersulfate, basischer Eisensulfate oder anderer Metallsalze stabilisiert wurden. Diese Art von Emulsionen wird auch "Pickering-Emulsionen" genannt. Grundlegende Untersuchungen zeigten, dass ein Charakteristikum für Pickering-Emulsionen ist, dass Feststoffpartikel an der Grenzfläche zwischen den beiden flüssigen Phasen angeordnet sind und dort eine Barriere gegen die Koaleszenz der dispersen Phase bilden.

Häufig zeigen derartige Feststoff-stabiliserte Emulsionen, wie sie z.B. in EP 987008 beschrieben wurden, jedoch eine hohe Viskosität und/oder eine große Tendenz zur Separation, d.h. zum Aufrahmen bzw. Sedimentieren der dispersen Phase.

Aufgabe der Erfindung war es, die Nachteile des Stands der Technik zu überwinden, insbesondere niedrigviskose und sedimentationsstabile Emulsionen mit kleinem Partikeldurchmesser der dispersen Phase zur Verfügung zu stellen.

Die Aufgabe wird durch die Erfindung gelöst.

Gegenstand der Erfindung sind Emulsionen vom Typ Wasser-in-Öl (W/O) bzw. Öl-in-Wasser (O/W) enthaltend:

- eine Ölphase (Phase A), enthaltend eine, gegebenenfalls mehrere, weitgehend wasserunlösliche Komponente(n),
- eine Wasserphase (Phase B), die gegebenenfalls weitere wasserlösliche Komponenten wie Salze oder organische Verbindungen wie Alkohole, Carbonsäuren oder andere Verbindungen enthalten kann
- an der Grenzfläche Öl-Wasser angeordnete pyrogene Kieselsäure, die in der Weise teilsilyliert ist, dass der Gehalt an nicht-silylierten Oberflächensilanolgruppen an der Siliciumdioxidoberfläche sich zwischen maximal 95% und minimal 5% des Ausgangs-Siliciumdioxids bewegt, gleichbedeutend mit 1,7 bis 0,1 SiOH-Gruppen pro $nm^2$ Kieselsäureoberfläche, der Dispersions-Anteil der Oberflächenenergie gamma-s-D 30 bis 80 $mJ/m^2$ beträgt, sowie die spezifische BET-Oberfläche einen Wert von 30 bis 500 $m^2/g$ aufweist,
- und gegebenenfalls weitere Stoffe, wie Pigmente oder Konservierungsmittel,

wobei die Emulsionen eine mittlere Partikelgröße der dispersen Phase, d.h. einen mittleren Tropfendurchmesser, gemessen mittels Laserbeugung von 0,5 $\mu$m bis 500 $\mu$m aufweist, die Emulsionen niedrigviskos sind, wobei niedrigviskos bedeutet, dass die Emulsionen relative Viskositäten $\eta_r$ im Bereich von 1 bis $10^6$ aufweisen, wobei die relative Viskosität als der Quotient $\eta_{rel} = \eta/\eta_0$ der gemessenen Viskosität der Emulsion $\eta$, gemessen bei 25 °C und einer Scherrate D = 10 $s^{-1}$, dividiert durch die Viskosität der reinen homogenen Phase $\eta_0$ definiert ist und die relative Viskosität $\eta_{rel}$ der Emulsion der Formel $\eta_{rel} = (1-\Phi/0,74)^{-([\eta]*0,74)}$ gehorcht, wobei $\Phi$ das Phasenvolumen der dispersen Phase und $[\eta]$ ein Formfaktor ist, der für die erfindungsgemäßen Emulsionen in einem Bereich von 2,5 bis 10 liegt.

Dabei war erstaunlich und für den Fachmann in keiner Weise vorherzusehen, dass durch die Verwendung von sinteraggregierter pyrogener Kieselsäure als partikulärer Emulgator niedrigviskose und sedimentationsstabile Emulsionen mit kleinem Partikeldurchmesser der dispersen Phase erhältlich sind. Dies ist insofern erstaunlich, als sinteraggregierte pyrogene Kieselsäure normalerweise als rheologisches Additiv zur Erhöhung der Viskosität flüssiger Medien eingesetzt wird.

Sinteraggregate sind hier Sekundärstrukturen gemäß DIN 53206, die unter Scherbedingungen wie sie üblicherweise beim Dispergieren von Füllstoffen in flüssigen Medien wie z.B. lösungsmittelhaltigen oder lösungsmittelfreien Kleb- oder Beschichtungsstoffen auftreten, permanent sind, d.h. sich nicht in ihre Primärpartikel zerteilen lassen. Dies läßt sich beispielsweise an TEM-Aufnahmen gehärteter Kieselsäure-Bindemittel-Dispersionen nachweisen, die nur Aggregat-Strukturen aber keine isolierten Primärpartikel aufweisen.

Partikuläre Systeme bestehend aus Sinteraggregaten sind ferner dadurch charakterisiert, das der bei der Partikelgrößenbestimmung mittels quasielastischer Lichtstreuung erhaltene hydrodynamische Äquivalentdurchmesser mindestens um dem Faktor 2 größer ist als der gemäß der Formel $a = 6/A_{BET} * d$ rechnerisch erhältliche Durchmesser der Primärpartikel, wobei $A_{BET}$ die mittels Stickstoffadsorption gemäß DIN 66131 gemessene spezifische BET-Oberfläche und d die Dichte der Primärpartikel ist.

Sinteraggregierte Systeme sind ferner dadurch charakterisiert, dass die fraktale Dimension df der Masse vorzugsweise kleiner 2,7 ist, wobei die fraktale Dimension df definiert ist als Masse ist proportional zum Radius R hoch df. Die fraktale Dimension der Masse läßt sich beispielsweise mittels Röntgen- oder Neutronen-Kleinwinkel-Streuung bestimmen.

Bevorzugt sind die erfindungsgemäßen Emulsionen im wesentlichen frei von herkömmlichen, bei Raumtemperatur und dem Druck der umgebenden Atmosphäre nicht partikulären flüssigen und festen, rein organischen oberflächenaktiven Substanzen, wie nichtionischen, kationischen und anionischen Emulgatoren.

Nicht-partikuläre Emulgatoren heißt hier keine Partikel und Kolloide, sondern Moleküle und Polymere, folgend der Definition von Molekülen, Polymeren, Kolloide und Partikel wie gegeben in 'Dispersionen und Emulsionen', G. Lagaly, O. Schulz, R. Zindel, Steinkopff, Darmstadt 1997, ISBN 3-7985-1087-3, S. 14.

Im allgemeinen weisen diese organischen Emulgatoren eine Größe kleiner 1 nm, eine Molmasse < 10000 g/mol, einen Kohlenstoffgehalt > 50 Gew.-%, bestimmbar durch Elementaranalyse, sowie eine Mohs'sche Härte kleiner als 1 auf.

Zugleich weisen die Emulgatoren, von denen die erfindungsgemäßen Emulsionen vorzugsweise im wesentlichen frei sind, zumeist eine Löslichkeit in Wasser bei 20 °C und dem Druck der umgebenden Atmosphäre, also 900 bis 1100 hPa, homogen oder in Micellen-Form von größer 1 Gew.-% auf.

Die erfindungsgemäßen Emulsionen können solche oberflächenaktive Substanzen bis zu einer maximalen Konzentration von kleiner als 0,1 mal, bevorzugt kleiner als 0,01 mal, besonders bevorzugt kleiner als 0,001 mal, insbesondere kleiner als 0,0001 mal der kritischen Micellkonzentration dieser oberflächenaktiven Stoffe in der Wasserphase enthalten; dies entspricht einer Konzentration dieser oberflächenaktiven Substanzen, bezogen auf das Gesamtgewicht der erfindungsgemäßen Emulsion von kleiner 10 Gew.-%, bevorzugt kleiner 2 Gew.-%, besonders bevorzugt kleiner 1 Gew.-%, insbesondere 0 Gew.-%.

Die erfindungsgemäße Emulsion enthält eine Ölphase (Phase A). Phase A enthält eine, gegebenenfalls mehrere, weitgehend wasserunlösliche Komponente(n). Weitgehend wasserunlöslich bedeutet hier, dass die Löslichkeit der Komponenten in Wasser alleine oder als Mischung kleiner 10 g/100 g Wasser, bevorzugt kleiner 1 g/100 g Wasser, besonders bevorzugt kleiner 0,1 g/100 g Wasser ist, gemessen bei 20 °C und dem Druck der umgebenden Atmosphäre, also 900 bis 1100 hPa ist.

Bei der erfindungsgemäßen Emulsion beträgt die Viskosität der Phase A, gemessen bei 20 °C und einem Schergefälle von 10 $s^{-1}$, 0,1 bis 1000000 mPa·s, bevorzugt 0,1 bis 500000 mPa·s, besonders bevorzugt 0,2 bis 100000 mPa·s.

Bei der erfindungsgemäßen Emulsion kann die Phase A vorzugsweise mehrere Komponenten enthalten. Bei den Einzelkomponenten kann es sich dabei sowohl um bei 20 °C flüssige Stoffe als auch um Feststoffe handeln, wobei die Gesamtmischung der Einzelkomponenten die oben genannte Viskosität aufweist. Bevorzugt, jedoch nicht notwendigerweise, handelt es sich bei einer mehrkomponentigen Phase A um eine echte Lösung, d.h. um eine homogene Phase bei der keine weiteren Phasengrenzflächen auftreten.

Beispiele für weitgehend wasserunlösliche Komponenten, wie sie die Phase A einer erfindungsgemäßen Emulsion bilden oder in ihr enthalten sein können, sind aliphatische und aromatische Kohlenwasserstoffe, Alkohole, Aldehyde, Ketone, Ether, Ester, Amine, Carbonsäuren und deren Derivate, Mercaptane, Thioether, oligomere bzw. polymere Verbindungen wie Polyolefine, wie Polystyrole, Polypropylene oder Polyethylene, gesättigte oder ungesättigte Polyester wie z.B. Polykondensate aus Phthalsäuren und 1,2-Propandiolen bzw. Poly-co-kondensate aus Phthalsäuren, 1,2-Propandiolen und Malensäuren gegebenenfalls gelöst in Reaktivverdünnern wie Styrolen, Polyether, Polyepoxide, bzw. deren monomeren oder oligomeren Vorstufen, wie Alkylenbisglycidylether, wie

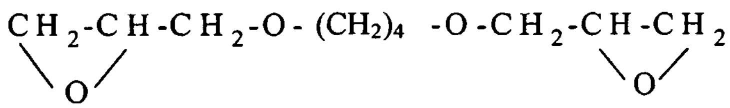

Bisphenol A basierende Diglycidylether, wie

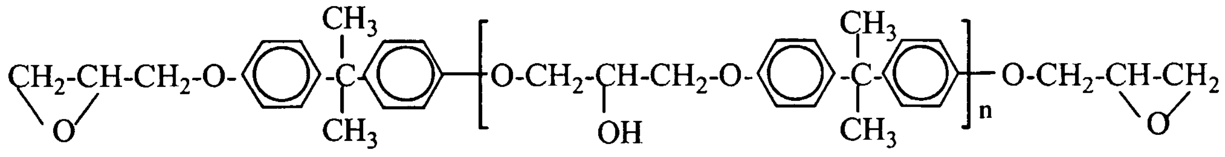

mit n bevorzugt von 0 bis 10, besonders bevorzugt 0 bis 5, Epoxy-Novolac-Harze, wie solche der Formel

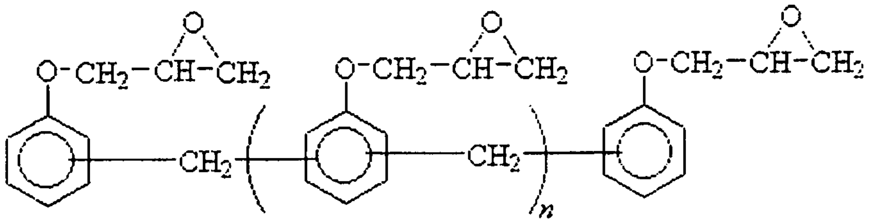

bifunktionelle Epoxyverbindungen, wie

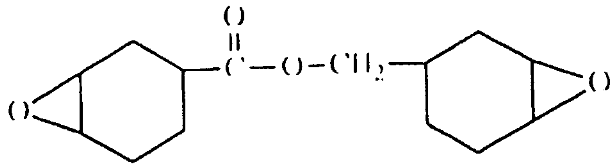

trifunktionelle Epoxyverbindungen, wie

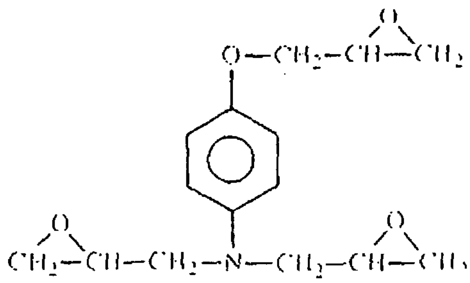

tetrafunktionelle Epoxyverbindungen, wie

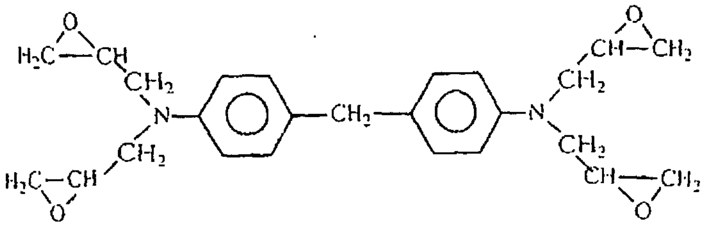

Polyurethane bzw. deren monomeren oder oligomeren Vorstufen wie z.B. Polyetherpolyole, Polyacrylatpolyole, Polyesterpolyole, multifunktionelle Isocyanate wie Hexandiisocyanat, Toluendiisocyanat, Diphenylmethandiisocyanat bzw. mit blockierenden Schutzgruppen versehene Isocyanate wie Hydroxylamine oder Malonesterderivate, komplexe organische Verbindungen wie künstliche bzw. natürliche pharmazeutische oder kosmetische Wirkstoffe, Farbstoffe, elementorganische Verbindungen wie Organosiliziumverbindungen, wie Organo(poly)silane, Organo(poly)siloxane, Organo (poly)silazane und Organo(poly)silcarbane, oder Übergangsmetallverbindungen. Gegebenenfalls kann Phase A ölbenetzbare Partikel wie Pigmente, Füllstoffe oder rheologische Additive enthalten.

Die erfindungsgemäße Emulsion enthält ferner eine wässrige Phase (Phase B). Phase B kann neben Wasser weitere Komponenten enthalten, wie vorzugsweise Säuren, Basen, Salze, wasserlösliche organische Verbindungen, wie Alkohole, Carbonsäuren und deren Derivate, Amine oder andere organische Verbindungen, polymere oder oligomere Verbindungen wie Polyole oder Polyamine bzw. Polyamidoamine, komplexe wasserlösliche organische Verbindungen, wie künstliche bzw. natürliche pharmazeutische oder kosmetische Wirkstoffe, Farbstoffe, elementorganische Verbindungen wie wasserlösliche Organosiliziumverbindungen oder wasserlösliche Übergangsmetallverbindungen. Gegebenenfalls kann Phase B wasserbenetzbare Partikel wie Pigmente, Füllstoffe oder rheologische Additive enthalten.

Die erfindungsgemäßen Emulsionen enthalten an der Grenzfläche Öl-Wasser angeordnete Sinteraggregate geeigneter pyrogener Kieselsäuren. Bei den erfindungsgemäß eingesetzten Sinteraggregaten handelt es sich um zum Teil mit Wasser benetzbare Sinteraggregate, d.h. die nicht vollständig mit Wasser benetzbar sind und nicht vollständig Wasser-unbenetzbar sind.

Bei den erfindungsgemäß eingesetzten Sinteraggregaten handelt es sich um bei Raumtemperatur und dem Druck der umgebenden Atmosphäre, also zwischen 900 und 1100 hPa, feste Sinteraggregate.

Die erfindungsgemäß eingesetzten Sinteraggregate weisen bevorzugt eine Löslichkeit in Wasser bei pH 7,33 und einem Elektrolythintergrund von 0,11 Mol und einer Temperatur von 37°C von kleiner 0,1 g/l, besonders bevorzugt von kleiner als 0,05 g/l, auf, bei dem Druck der umgebenden Atmosphäre, also zwischen 900 und 1100 hPa.

Bevorzugt weisen die erfindungsgemäß eingesetzten Sinteraggregate einen mittleren hydrodynamischen Äquivalenzdurchmesser $D_h$ größer 1 nm, vorzugsweise von 1 bis 5000 nm, bevorzugt von 10 bis 1000 nm, besonders bevorzugt von 100 bis 600 nm, ganz besonders bevorzugt 200 nm bis 500 nm, speziell ausgewählt von 210 nm bis 450 nm auf, jeweils gemessen bevorzugt mittels dynamischer Lichtstreuung.

Dies bedeutet, dass der für die Ausbildung einer Partikelschicht in der Öl-Wasser-Grenzfläche relevante Kollisionsradius $R_c$ der Sinteraggregate größer 0,8 nm, vorzugsweise 0,8 bis 4000 nm, bevorzugt 8 bis 850 nm, besonders bevorzugt 80 bis 500 nm, ganz besonders bevorzugt 170 nm bis 375 nm ist. Der Kollisionsradius ist dabei der Radius der kleinsten Kugel, die gerade noch alle Bestandteile eines Aggregates einschließt, wobei sich der Kollisionsradius $R_c$ ergibt aus der Gleichung $R_c = [R_h^2/0,76 + 2,63 * R_h^2/d_f]^{0,5}$ wie gegeben in R. de Rooij, A. A. Potanin, D. van den Ende, J. Mellema, J. Chem. Phys. 1993, 99, 9213, wobei der hydrodynamische Äquivalenzradius $R_h$ erhalten wird aus hydrodynamische Äquivalenzdurchmesser dividiert durch 2 und die fraktale Dimension der Masse $d_f$ einen Wert von 1,8 aufweist.

Bevorzugt sind die erfindungsgemäß eingesetzten Sinteraggregate ferner dadurch charakterisiert, dass bei der Partikelgrößenbestimmung mittels quasielastischer Lichtstreuung der hydrodynamische Äquivalentdurchmesser mindestens um dem Faktor 2, bevorzugt um den Faktor 5 bis 50, besonders bevorzugt um den Faktor 7 bis 25, ganz besonders bevorzugt um den Faktor 7,5 bis 16,5, jeweils bezogen auf eine spezifische Oberfläche von 100 $m^2/g$, bei kleinerer bzw. größerer Oberfläche verkleinert bzw. vergrößert sich der Faktor entsprechend linear, größer ist als der gemäß der Formel $a = 6/A_{BET} * d$ rechnerisch erhältliche Durchmesser der Primärpartikel, wobei $A_{BET}$ die mittels Stickstoffadsorption gemäß DIN 66131 gemessene spezifische BET-Oberfläche und d die Dichte der Primärpartikel ist.

Bevorzugt weisen die erfindungsgemäß eingesetzten Sinteraggregate eine Molmasse größer 10 000 g/Mol, besonders bevorzugt eine Molmasse von 50 000 bis 50 000 000 g/Mol, insbesondere von 100 000 bis 10 000 000 g/Mol, auf, jeweils gemessen bevorzugt mittels statischer Lichtstreuung.

Bevorzugt weisen die erfindungsgemäß eingesetzten Sinteraggregate eine spezifische BET-Oberfläche von 30 bis 500 $m^2/g$, besonders bevorzugt 80 bis 300 $m^2/g$, auf. Die BET-Oberfläche wird nach bekannten Verfahren gemessen, bevorzugt gemäß Deutscher Industrie Norm DIN 66131 und DIN 66132.

Bevorzugt weisen die erfindungsgemäß eingesetzten Sinteraggregate einen Kohlenstoffgehalt von kleiner 50 Gewichtsprozent auf.

Bevorzugt weisen die erfindungsgemäß eingesetzten Sinteraggregate eine Mohs'sche Härte größer als 1, besonders bevorzugt größer als 4, auf.

Vorzugsweise weisen die Sinteraggregate eine Oberflächenenergie gamma von 30 bis 72,5 mJ/m$^2$, bei einer Temperatur von 25°C und dem Druck der umgebenden Atmosphäre, also zwischen 900 und 1100 hPa, auf.

Die erfindungsgemäß eingesetzten Kieselsäure-Sinteraggregate weisen einen Dispersions-Anteil der Oberflächenenergie gamma-s-D von 30 bis 80 mJ/m$^2$, bevorzugt 35 bis 70 mJ/m$^2$, besonders besonders bevorzugt 40 bis 70 mJ/m$^2$, bei einer Temperatur von 25°C und dem Druck der umgebenden Atmosphäre, also zwischen 900 und 1100 hPa, auf. Gemessen wird der Dispersions-Anteil der Oberflächenenergie gamma-s-D zum
Beispiel gemäß "Inverse Gaschromatography"-"Characterisation of Polymers and other Materials", 391 ACS Symposium Series, D R Lloyd, Th C Ward, H P Schreiber, Chapter 18, Seiten 248-261, ACS, Washington DC 1989, ISBN 0-8412-1610-X.

Die bevorzugte Ausgangs-Kieselsäure, aus der die in den erfindungsgemäßen Emulsionen eingesetzte, zum Teil mit Wasser benetzbare Kieselsäure hergestellt wird, kann auf beliebige an sich bekannte Weise, wie beispielsweise in einer Flammenreaktion aus Halogensiliciumverbindungen hergestellt werden, z.B. aus Siliciumtetrachlorid oder Halogen-Organosiliciumverbindungen, wie Methylchlorsilanen, wie Methyltrichlorsilan, oder Hydrogenchlorsilanen, wie Hydrogentrichlorsilan oder anderen Hydrogenmethylchlorsilanen, wie Hydrogenmethyldichlorsilan oder Alkylchlorsilanen, auch im Gemisch mit Kohlenwasserstoffen, oder beliebigen versprühbaren und, bevorzugt, verflüchtigbaren Gemischen aus Organosiliciumverbindungen, wie genannt, und Kohlenwasserstoffen, wobei es sich bei der Flamme um eine Wasserstoff-Sauerstoff-Flamme oder auch eine Kohlenmonoxid-Sauerstoff-Flamme handeln kann. Die Herstellung der Kieselsäure kann dabei wahlweise mit und ohne zusätzlichem Zusatz von Wasser erfolgen, zum Beispiel im Schritt der Reinigung; bevorzugt ist kein Zusatz von Wasser.

Bevorzugt werden zur Herstellung der erfindungsgemäßen Emulsionen als Kieselsäure-Sinteraggregate teilhydrophobierte, besonders bevorzugt teilsilylierte, Kieselsäure-Sinteraggregate eingesetzt.

Teilweise silyliert bedeutet hier, dass weder die gesamte Kieselsäure-Oberfläche unsilyliert ist, noch dass die gesamte Kieselsäure-Oberfläche silyliert ist.

Der Bedeckungsgrad $\tau$ der Oberfläche der Kieselsäure-Sinteraggregate mit Silyliermittelresten ist dabei, bezogen auf die Gesamt-Partikel-Oberfläche, 5 bis 95 %, besonders bevorzugt 10 bis 90 %, insbesondere 15 bis 75 %.

Die Bedeckung mit Silyliermittel kann dabei beispielsweise mittels Elementaranalyse, wie z.B. über den Kohlenstoffgehalt, ermittelt werden oder durch Bestimmung des Rest-Gehaltes an reaktiven Oberflächen-Silanol-Gruppen der Kieselsäure-Sinteraggregate.

Teilsilylierung bedeutet ferner, dass der Gehalt an nicht-silylierten Oberflächensilanolgruppen an der Siliciumdioxidoberfläche sich zwischen maximal 95 % und minimal 5 %, besonders bevorzugt von 90 bis 10 %, insbesondere 85 bis 25 %, der Silanolgruppen des Ausgangs-Siliciumdioxids bewegt.

Dies bedeutet, dass die Dichte der Oberflächensilanolgruppen SiOH sich zwischen minimal 0,1 und maximal 1,7, bevorzugt von 0,2 bis 1,6, besonders bevorzugt von 0,45 bis 1,55, SiOH pro nM$^2$ Partikeloberfläche bewegt.

Für ein Ausgangs-Siliciumdioxid von 200 m$^2$/g spezifischer Oberfläche, das zur Silylierung herangezogen werden kann, bedeutet dies vorzugsweise zwischen minimal 0,03 mMol/g SiOH und maximal 0,57 mMol/g SiOH, bevorzugt 0,06 bis 0,54 mMol/g SiOH, besonders bevorzugt 0,15 bis 0,51 mMol/g SiOH; für ein Siliciumdioxid mit geringerer bzw. größerer Oberfläche bedeutet dies linear proportional mehr oder weniger Oberflächensilanolgruppen SiOH.

Die erfindungsgemäß eingesetzten Kieselsäuren weisen einen Kohlenstoffgehalt von 0,1 bis 20 Gew-%, bevorzugt 0,1 bis 15 Gew-%, besonders bevorzugt 0,1 bis 10 Gew-% auf.

Verfahren zur Teilhydrophobierung bzw. Teilsilylierung von Feststoffpartikel sind bereits bekannt.

Vorzugsweise weist die Ausgangs-Kieselsäure eine spezifische BET-Oberfläche von 25 bis 500 m$^2$/g auf. Die Ausgangs-Kieselsäure weist vorzugsweise Sinteraggregate (Definition nach DIN 53206) im Bereich von Durchmessern 200 bis 1000 nm auf,
wobei die Kieselsäure aus Sinteraggregaten aufgebaute Agglomerate (Definition nach DIN 53206) aufweist, die in Abhängigkeit von der äußeren Scherbelastung (z.B. Messbedingungen) Größen von 1 bis 500 $\mu$m aufweisen.

Vorzugsweise weist die Ausgangs-Kieselsäure eine fraktale Dimension der Oberfläche von vorzugsweise kleiner oder gleich 2,3 auf, wobei die fraktale Dimension der Oberfläche $D_s$ hierbei definiert ist als: Partikel-Oberfläche A ist proportional zum Partikel-Radius R hoch $D_s$. Vorzugsweise weist die Ausgangs-Kieselsäure eine Dichte an zugänglichen, d.h. einer chemischen Reaktion zugänglichen, Oberflächen-Silanolgruppen SiOH von bevorzugt 1,5 bis 2,5 SiOH pro nm$^2$ spezifischer Oberfläche, besonders bevorzugt 1,6 bis 2,0 SiOH pro nm$^2$, auf.

Zur Herstellung der erfindungsgemäß eingesetzten Kieselsäure-Sinteraggregate werden als Ausgangs-Kieselsäuren pyrogen hergestellte Kieselsäuren eingesetzt. Es können hydrophile Kieselsäuren eingesetzt werden, die frisch hergestellt direkt aus dem Brenner kommen, zwischengelagert oder bereits handelsüblich verpackt sind.

Als Ausgangs-Kieselsäuren können unverdichtete, mit Stampf- oder Klopfdichten kleiner 60 g/l, aber auch

verdichtete, mit Stampf- oder Klopfdichten größer 60 g/l, Kieselsäuren eingesetzt werden.

Als Ausgangs-Kieselsäuren können Gemische aus verschiedenen Kieselsäuren eingesetzt werden, so z.B. Mischungen aus Kieselsäuren unterschiedlicher BET-Oberfläche.

Bevorzugt können zur Silylierung von Kieselsäuren Organosiliciumverbindungen, wie z.B.

(i) Organosilane bzw. Organosilazane der Formel

$$R^1_dSiY_{4-d} \qquad (I)$$

und/oder deren Teilhydrolysate,
wobei
$R^1$ gleich oder verschieden sein kann und einen einwertigen, gegebenenfalls substituierten, gegebenenfalls einfach oder mehrfach ungesättigten, gegebenenfalls aromatischen Kohlenwasserstoffrest, mit 1 bis 24 Kohlenstoff-Atomen, der durch Sauerstoffatome unterbrochen sein kann, bedeutet, d gleich 1, 2 oder 3 bedeutet und
Y gleich oder verschieden sein kann und Halogenatom, einwertige Si-N-gebundene Stickstoffreste, an den ein weiterer Silylrest gebunden sein kann, $-OR^2$ oder $-OC(O)OR^2$ bedeutet, wobei $R^2$ gleich Wasserstoffatom oder ein einwertiger, gegebenenfalls substituierter, gegebenenfalls einfach oder mehrfach ungesättigten Kohlenwasserstoffrest, der durch Sauerstoffatome unterbrochen sein kann, bedeutet,
oder
(ii) lineare, verzweigte oder cyclische Organosiloxane aus Einheiten der Formel

$$R^3_e(OR^4)_fSiO_{(4-e-f)/2} \qquad (II),$$

wobei
$R^3$ gleich oder verschieden sein kann und eine der oben für $R^1$ angegebenen Bedeutungen hat,
$R^4$ gleich oder verschieden sein kann und eine für $R^3$ angegebene Bedeutung hat,
e 0, 1, 2 oder 3 ist und
f 0, 1, 2, 3 ist, mit der Maßgabe dass die Summe $e+f \leq 3$ ist, oder
Gemische aus (i) und (ii)
eingesetzt werden.

Bei den Organosiliciumverbindungen, die zur Silylierung der Kieselsäuren eingesetzt werden können, kann es sich beispielsweise um Gemische aus Silanen oder Silazanen der Formel (I) handeln, wobei solche aus Methyl-Chlorsilanen einerseits oder Alkoxysilanen und gegebenenfalls Disilazanen andererseits bevorzugt sind.

Beispiele für $R^1$ in Formel I sind vorzugsweise der Methyl-, Octyl-, Phenyl- und Vinylrest, besonders bevorzugt ist der Methylrest.

Beispiele für $R^2$ sind vorzugsweise der Methyl-, der Ethyl-, der Propyl und der Octylrest, wobei der Methyl- und der Ethylrest bevorzugt sind.

Beispiele für Organosilane der Formel (I) sind Alkylchlorsilane, wie Methyltrichlorsilan, Dimethyldichlorsilan, Trimethylchlorsilan, Octylmethyldichlorsilan, Octyltrichlorsilan, Octadecylmethyldichlorsilan und Octadecyltrichlorsilan, Methylmethoxysilane, wie Methyltrimethoxysilan, Dimethyldimethoxysilan und Trimethylmethoxysilan, Methylethoxysilane, wie Methyltriethoxysilan, Dimethyldiethoxysilan und Trimethylethoxysilan, Methylacetoxysilane, wie Methyltriacetoxysilan, Dimethyldiacethoxysilan und Trimethylacetoxysilan, Phenylsilane, wie Phenyltrichlorsilan, Phenylmethyldichlorsilan, Phenyldimethylchlorsilan, Phenyltrimethoxysilan, Phenylmethyldimethoxysilan, Phenyldimethylmethoxysilan, Phenyltriethoxysilan, Phenylmethyldiethoxysilan und Phenyldimethylethoxysilan,Vinylsilane, wie Vinyltrichlorsilan, Vinylmethyldichlorsilan, Vinyldimethylchlorsilan, Vinyltrimethoxysilan, Vinylmethyldimethoxysilan, Vinyldimethylmethoxysilan, Vinyltriethoxysilan, Vinylmethyldiethoxysilan und Vinyldimethylethoxysilan, Disilazane wie Hexarnethyldisilazan, Divinyltetramethyldisilazan und Bis(3,3-trifluorpropyl)tetramethyldisilazan, Cyclosilazane wie Octamethylcyclotetrasilazan, und Silanole wie Trimethylsilanol.

Bevorzugt ist Methyltrichlorsilan, Dimethyldichlorsilan und Trimethylchlorsilan oder Hexamethyldisilazan.

Beispiele für Organosiloxane der Formel (II) sind lineare oder cyclische Dialkylsiloxane mit einer mittleren Anzahl an Dialkylsiloxyeinheiten von größer als 3. Die Dialkylsiloxane sind bevorzugt Dimethylsiloxane. Besonders bevorzugt sind lineare Polydimethylsiloxane mit folgenden Endgruppen: Trimethylsiloxy-, Dimethylhydroxysiloxy-, Dimethylchlorsiloxy-, Methyldichlorsiloxy-, Dimethylmethoxysiloxy-, Methyldimethoxysiloxy-, Dimethylethoxysiloxy-, Methyldiethoxysiloxy-, Dimethylacetoxysiloxy-, Methyldiacetoxysiloxy- und Dimethylhydroxysiloxygruppen, insbesondere mit Trimethylsiloxy- oder Dimethylhydroxysiloxyendgruppen.

Bevorzugt haben die genannten Polydimethylsiloxane eine Viskosität bei 25°C von 2 bis 100 mPa·s.

Weitere Beispiele für Organosiloxane sind Siliconharze, im besonderen solche, die als Alkylgruppen Methylgruppen enthalten, wobei es sich besonders bevorzugt um solche handelt, die $R^3{}_3SiO_{1/2}$ und $SiO_{4/2}$-Einheiten enthalten oder solche, die $R^3SiO_{3/2}$ und gegebenenfalls $R^3{}_2SiO_{2/2}$-Einheiten enthalten,
wobei $R^3$ eine der oben genannten Bedeutungen hat.

Bevorzugt haben die genannten Siliconharze aus Einheiten der Formel (II) eine Viskosität bei 25°C von 500 bis 5000 $mm^2/s$.

Bei Siliconharzen mit einer Viskosität von größer als 1000 $mm^2/s$ bei 25°C sind solche bevorzugt, die sich in einem technisch gut handhabbaren Lösungsmittel, wie vorzugsweise Alkohole wie Methanol, Ethanol, iso-Propanol, Ether wie Diethylether, Tetrahydrofuran, Siloxane wie Hexamethyldisiloxan, Alkane wie Cyclohexan oder n-Octan, Aromaten wie Toluol oder Xylol, mit einer Konzentration über 10 Gew.-% und einer Mischungsviskosität kleiner als 1000 $mm^2/s$ bei einer Temperatur von 25°C und dem Druck der umgebenden Atmosphäre lösen lassen.

Unter den festen Organosiloxanen sind solche bevorzugt, die sich in einem technisch handhabbaren Lösungsmittel (wie oben definiert) mit einer Konzentration größer als 10 Gew.% und einer Mischungsviskosität kleiner als 1000 $mm^2/s$ bei einer Temperatur von 25°C lösen.

Die Hydrophobierung und Silylierung, die zur Herstellung der erfindungsgemäß eingesetzten Kieselsäure-Sinteraggregate bevorzugt durchgeführt wird, kann als diskontinuierliche Reaktion, d.h. im Batch-Verfahren, oder als kontinuierliche Reaktion durchgeführt werden, wobei die kontinuierliche Reaktion bevorzugt ist.

Die Hydrophobierung und Silylierung kann in einem Schritt realisiert werden oder in 2 oder 3 aufeinander folgenden Schritten. Das heißt, der Reaktion kann eine Beladung (Physisorption des Silyliermittels) vorgeschaltet sowie der Reaktion vorzugsweise ein Reinigungsschritt nachgeschaltet sein. Bevorzugt sind 3 sukzessive Schritte: (1) Beladung - (2) Reaktion - (3) Reinigung.

Die Beladungstemperatur liegt bei vorzugsweise -30 bis 350°C, bevorzugt 20 bis 120°C.

Die Reaktionstemperaturen reichen vorzugsweise von 0 bis 400°C, besonders bevorzugt von 20 bis 330°C.

Die Reaktionszeiten dauern vorzugsweise von 1 Minute bis 24 Stunden, bevorzugt 30 Minuten bis 4 Stunden.

Der Reaktionsdruck liegt vorzugsweise im Bereich Normaldruck, d.h. zwischen 900 und 1100 hPa.

Die Reinigungstemperatur reicht vorzugsweise von 80 bis 400°C.

Eine effektive Bewegung und Durchmischung von Kieselsäure und Silyliermittel während Schritte (1) Beladung, (2) Reaktion und Reinigung (3) ist notwendig. Dies erfolgt bevorzugt durch mechanische oder gasgetragene Fluidisierung. Eine gasgetragene Fluidisierung kann durch alle inerten Gase erfolgen, die nicht zu Nebenreaktionen, Abbaureaktionen, Oxidationsvorgängen und Flammen- und Explosionserscheinungen führen. Die Leerrohrgasgeschwindigkeit ist hierbei von 0,05 bis 5 cm/s, besonders bevorzugt von 0,05 bis 1 cm/s. Mechanische Fluidisierung kann durch Flügelrührer, Ankerrührer und sonstige geeignete Rührorgane erfolgen.

In einer besonders bevorzugten Ausführung wird nur die Gasmenge zugeführt, die zur Aufrechterhaltung einer sauerstoffarmen Atmosphäre ausreicht, bevorzugt weniger als 5 Vol.-%, die Fluidisierung erfolgt dann rein mechanisch.

Die Reaktion wird bevorzugt in einer Atmosphäre durchgeführt, die nicht zur Oxidation der silylierten Kieselsäure führt, d.h. bevorzugt weniger als 10 Vol.-% Sauerstoff, besonders bevorzugt sind weniger als 2,5 Vol.-%, wobei beste Ergebnisse bei weniger als 1 Vol.-% Sauerstoff erzielt werden.

Es erfolgt ein effektives Einbringen der Silyliermittel in die Kieselsäure. Handelt es sich bei den Silyliermitteln bei Applikationstemperatur um flüssige Verbindungen, werden bevorzugt effektive Verdüsungstechniken eingesetzt. Verdüsen in 1-Stoffdüsen unter Druck (5 bis 20 bar), Versprühen in 2-Stoffdüsen unter Druck (Gas und Flüssigkeit 2 bis 20 bar), Feinstverteilen mit Atomizern, etc.

Bevorzugt wird das Silyliermittel als feinstverteiltes Aerosol zugefügt, wobei das Aerosol eine Sinkgeschwindigkeit von vorzugsweise 0,1 bis 20 cm/s und eine Tropfengröße mit einem aerodynamischen Äquivalentdurchmesser von 5 bis 25 $\mu$m aufweist.

Wahlweise können vorzugsweise protische Lösemittel hinzugefügt werden, wie flüssige oder verdampfbare Alkohole oder Wasser; typische Alkohole sind iso-Propanol, Ethanol und Methanol. Es können auch Gemische der oben genannten protischen Lösemittel zugefügt werden. Bevorzugt werden keine protischen Lösemittel zugesetzt.

Wahlweise können vorzugsweise saure oder basische Katalysatoren zugesetzt werden. Diese Katalysatoren können basischen Charakters sein, im Sinne einer Lewis Base oder einer Brönsted Base, wie Ammoniak, oder sauren Charakters sein, im Sinne einer Lewis Säure oder einer Brönsted Säure, wie Chlorwasserstoff. Falls Katalysatoren eingesetzt werden, handelt es sich bevorzugt um Spuren, d.h. weniger als 1000 ppm. Besonders bevorzugt werden keine Katalysatoren zugesetzt.

Der Reinigungsschritt ist durch Bewegung gekennzeichnet, wobei langsame Bewegung und geringes Durchmischen bevorzugt ist.

Der Reinigungsschritt ist weiterhin durch erhöhten Gaseintrag gekennzeichnet, entsprechend einer Leerrohrgasgeschwindigkeit von 0,001 bis 10 cm/s.

Zusätzlich kann der Reinigungsschritt ein Mischen mit mechanischen Rührorganen beinhalten. Die Rührorgane werden dabei so eingestellt und bewegt, dass bevorzugt Mischen und Fluidisieren, jedoch nicht völlige Verwirbelung eintritt.

Zusätzlich können während des Silylierschrittes Verfahren zur mechanischen Verdichtung eingesetzt werden, wie zum Beispiel Presswalzen, Kugelmühlen, Kollergänge, Schraubenverdichter und Brikettierer.

Zusätzlich können vor, während oder nach dem Silylierungsschritt Verfahren zur Desagglomerierung der Kieselsäure eingesetzt werden, wie Stiftmühlen oder Vorrichtungen zur Mahlsichtung und/oder Verfahren zur mechanischen Verdichtung der Kieselsäure, wie zum Beispiel Presswalzen, oder Verdichten durch Absaugen des Luft- oder Gasinhaltes durch geeignete Vakuummethoden oder andere Verfahren zur mechanischen Verdichtung wie zum Beispiel Presswalzen, Kugelmühlen, Kollergänge, Schraubenverdichter und Brikettierer.

Die Herstellung der erfindungsgemäßen Kieselsäure-Sinteraggregate kann auch in-situ bei der Herstellung der erfindungsgemäßen Emulsionen erfolgen.

Die erfindungsgemäßen Emulsionen enthalten Kieselsäure-Sinteraggregate in Mengen von bevorzugt 0,1 bis 50 Gewichtsteilen, besonders bevorzugt 1 bis 15 Gewichtsteilen, insbesondere 2 bis 10 Gewichtsteilen, bezogen auf 100 Gewichtsteile Gesamtemulsion.

Bei den erfindungsgemäßen Emulsionen kann der Volumenbruch $\Phi_o$ der Ölphase definiert als $\Phi_o$ = Volumen Ölphase / (Volumen Ölphase + Volumen Wasserphase) vorzugsweise 0,1 bis 0,9 bevorzugt 0,2 bis 0,8, besonders bevorzugt 0,3 bis 0,7, insbesondere 0,4 bis 0,6 betragen.

Bei den erfindungsgemäßen Emulsionen kann der Volumenbruch $\Phi_w$ der Wasserphase definiert als $\Phi_w$ = Volumen Wasserphase / (Volumen Ölphase + Volumen Wasserphase) vorzugsweise 0,1 bis 0,9, bevorzugt 0,2 bis 0,8, besonders bevorzugt 0,3 bis 0,7, insbesondere 0,4 bis 0,6 betragen.

Die erfindungsgemäßen Emulsionen sind insbesondere dadurch charakterisiert, dass die mittlere Partikelgröße der dispersen Phase, d.h. der mittlere Tropfendurchmesser, gemessen mittels Laserbeugung, z.B. auf einem Laserbeugungsgerät der Fa. Sympatec in Küvettenmeßtechnik, 0,5 $\mu$m bis 500 $\mu$m, bevorzugt 0,7 $\mu$m bis 100 $\mu$m, besonders bevorzugt 0,7 $\mu$m bis 50 $\mu$m und ganz besonders bevorzugt 0,7 $\mu$m bis 10 $\mu$m beträgt.

Die erfindungsgemäßen sinteraggregatstabilisierten Emulsionen sind insbesondere dadurch charakterisiert, dass sie niedrigviskos sind. Niedrigviskos bedeutet hier, dass die erfindungsgemäßen Emulsionen relative Viskositäten $\eta_r$ im Bereich von 1 bis $10^6$, bevorzugt 1 bis $5*10^5$, besonders bevorzugt kleiner $10^5$ aufweisen. Die relative Viskosität ist dabei definiert als der Quotient $\eta_{rel} = \eta/\eta_o$ der gemessenen Viskosität der Emulsion $\eta$, gemessen bei 25 °C mit einem Kegel-Platte-System bei einem Meßspalt von 105 $\mu$m und einem Scherrate D = 10 $s^{-1}$, dividiert durch die Viskosität der reinen homogenen Phase $\eta_o$, gemessen bei 25 °C.

Die erfindungsgemäßen sinteraggregatstabilisierten Emulsionen sind ferner dadurch charakterisiert, dass die relative Viskosität $\eta_{rel}$ der Emulsion der Formel $\eta_{rel} = (1-\Phi/0,74)^{-([\eta]*0,74)}$ gehorcht. Dabei ist $\Phi$ das Phasenvolumen der dispersen Phase und $[\eta]$ ein Formfaktor, der für die erfindungsgemäßen Emulsionen in einem Bereich von 2,5 bis 10 liegt.

Die erfindungsgemäßen partikelstabilisierten Emulsionen zeichnen sich insbesondere dadurch aus, dass sie weitgehend stabil gegen Separation der dispersen Phase, d.h. weitgehend stabil gegen Aufrahmen bzw. Sedimentation der dispersen Phase sind. Weitgehend stabil gegen Separation bedeutet hier, dass das Volumen der dispersionsverarmten Phase weniger als 10% des Gesamtvolumens, bevorzugt weniger als 5% des Gesamtvolumens, besonders bevorzugt weniger als 1% des Gesamtvolumens beträgt.

Die Emulsionsstabilität wurde dabei mittels des im folgenden beschriebenen Stabilitätsanalysators untersucht.

Es handelt sich um einen Stabilitätsanalysator, der einen Flachbettscanner mit einem Probenhalter, um Messküvetten senkrecht zur Scannerlampe zu halten, einen gekippten Spiegel, der das Licht der Scannerlampe seitlich auf die Messküvetten lenkt und eine Auswertvorrichtung, um das empfangene Licht auszuwerten, aufweist.

Auf einem Flachbettscanner (z.B. HPScanJet 3300C der Fa. Hewlett-Packard) werden runde Glasküvetten mit einem äußeren Durchmesser von 12,5 mm und einer Höhe von 50 mm in einer Reihe angeordnet. Die Küvetten werden dabei durch einen temperierbaren Probenhalter (Abb. 1) gegen Verrutschen gesichert.

Siehe Figur 1: Temperierbare Probenhalter

Die Proben werden dabei auf dem Flachbettscanner parallel zur Längsseite des Scanners ausgerichtet, d.h. senkrecht zur Scannerlampe (s. Abb. 2).

Siehe Figur 2: Aufbau des Stabilitätsanalysators

Jeder Probenhalter enthält bis zu 12 Proben, insgesamt können pro Meßvorgang 24 Proben gleichzeitig untersucht werden.

Die Bilderzeugung erfolgt dabei in der Weise, dass durch einen gekippten Spiegel das Licht der Scannerlampe seitlich auf die Messküvetten gelenkt wird. Parallel dazu wird die Emulsion durch den Küvettenboden durchleuchtet (dicke Pfeile in Abb. 2). Das durch die Emulsion erzeugt Streulicht (gestrichelte Pfeile in Abb.2) wird dann über den Spiegel zurück zum Sensor des Flachbettscanners gelenkt. Der Lichtweg kann dabei durch Justierschrauben (aus Übersichtlichkeitsgründen nicht gezeigt) eingestellt werden. Zum Schutz des Scanners vor Verunreinigung und der Proben vor Wärme durch die Scannerlampe befindet sich auf dem Scannerfenster noch eine 2 mm Dicke Plexiglasscheibe.

Das empfangene Licht wird durch den Scanner digitalisiert und auf einem Computer in Form vorzugsweise

einer Bitmap-Datei mit 256 Graustufen gespeichert. Zur Ermittlung der Graustufe, d.h. der Streulichtintensität, in einer Höhe h vom Küvettenboden werden die Lichtintensitäten von 60 Pixeln einer Reihe gemäß der Formel $I_{av}(h)=\left(\sum_{i=1}^{60} I_i\right)/60$ gemittelt. $I_{av}(h)$ entspricht der

Graustufe in der Höhe h vom Küvettenboden. Bei einem Flachbettscanner mit 600 dpi wird die gesamte Streulichtintensitätsverteilung, wie sie z.B. in Figur 2 gezeigt wird, aus 980 Reihen mit jeweils 60 Pixeln erhalten.

Der Meß- und Auswertevorgang für 24 Probenküvetten beträgt dabei weniger als 2 min, d.h. weniger als 5 s pro Küvette.

Die Auswertung erfolgt nun in der Weise, dass die gemessene Streulichtintensität in Abhängigkeit von der Höhe gemessen vom Küvettenboden aufgetragen wird. Zur Bewertung der Stabilität einer Emulsion werden Messungen in definierten Abständen durchgeführt. Eine Auftragung von $\Delta I(z,t)$ - $I(z,t)$ - $I(z,0)$,
wobei $I(z,t)$ die Streulichtintensität in der Höhe z zum Zeitpunkt t und $I(z,0)$ die Streulichtintensität in der Höhe z zum Zeitpunkt 0 ist, liefert die Veränderung der Streulichtintensität mit der Zeit. Eine positive Veränderung bedeutet dabei eine höhere Konzentration an streuenden Teilchen und weist somit auf Separation der Emulsion hin, analog bedeutet eine zeitliche Abnahme der Streulichtintensität in einem bestimmten Volumenelement ein Verarmen dieses Elementes an streufähigen Teilchen.

In Figur 3 und 4 wird dies am Beispiel einer nicht stabilen Emulsion verdeutlicht.

Ein weitererGegenstand ist ein Verfahren zur Herstellung der Emulsionen, wobei in einem ersten Schritt eine hochkonzentrierte feinteilige Dispersion der entsprechenden Kieselsäure in der Flüssigkeit, die in der Emulsion die homogene Phase bildet hergestellt wird und in einem zweiten Schritt eine hochviskose Präemulsion hergestellt wird, welche die Gesamtmenge der dispersen Phase und die im ersten Schritt hergestellte hochkonzentrierte feinteilige Dispersion der Kieselsäure in der Flüssigkeit, die in der erfindungsgemäßen Emulsion die homogene Phase bildet aufweist, wobei das eingesetzte Volumen der Dispersion so bemessen ist, dass die gesamte Menge der benötigen sinteraggregierten Kieselsäure enthalten ist, und in einem dritten Schritt die restliche homogenen Phase langsam eindosiert wird.

Als wesentlich für das Erzielen der oben beschriebenen Eigenschaften der erfindungsgemäßen Emulsionen erwies sich dabei ein Emulgierverfahren, bei dem während der Emulgierung ein Zustand hoher Viskosität, im weiteren als 'steife Phase' bezeichnet, durchlaufen wird.

Im Einzelnen weist der Prozess zur Herstellung der erfindungsgemäßen Emulsionen folgende Einzelschritten auf:

- Herstellung einer hochkonzentrierten feinteiligen Dispersion der entsprechenden geeigneten Kieselsäure in der Flüssigkeit, die in der erfindungsgemäßen Emulsion die homogene Phase bildet.
- Herstellung einer hochviskosen Präemulsion, bestehend aus der hochkonzentrierten feinteiligen Dispersion der entsprechenden geeigneten Kieselsäure in der Flüssigkeit, die in der erfindungsgemäßen Emulsion die homogene Phase bildet, wobei das eingesetzte Volumen so bemessen ist, dass die gesamte Menge der benötigen sinteraggregierten Kieselsäure enthalten ist, und der Gesamtmenge der dispersen Phase
- langsames Eindosieren der restlichen homogenen Phase unter Scheren

Die Herstellung der hochkonzentrierten feindispersen Dispersion der entsprechenden geeigneten Kieselsäure in der Flüssigkeit, die in der erfindungsgemäßen Emulsion die homogene Phase bildet, kann dabei grundsätzlich gemäß den bekannten Verfahren zur Herstellung von Kieselsäuredispersionen erfolgen, wie das Einarbeiten mittels Rührorgane mit hoher Scherwirkung wie schnellaufende Rührer, schnellaufende Dissolver, Rotor-Stator-Systeme, Ultraschalldispergatoren oder Kugel- bzw. Perlmühlen.

Die Konzentration der Kieselsäure-Sinteraggregate in der Dispersion beträgt dabei zwischen 1 und 80 Gew. %, bevorzugt zwischen 10 und 60 Gew.%, besonders bevorzugt zwischen 10 und 40 Gew.% und ganz besonders bevorzugt zwischen 12 und 30 Gew.%.

Die Herstellung der hochviskosen Präemulsion kann grundsätzlich gemäß den bekannten Verfahren zur Herstellung von Emulsionen erfolgen, es zeigte sich jedoch, dass die im folgenden beschriebenen Verfahren besonders geeignet sind, die erfindungsgemäßen Emulsionen zu erhalten.

Verfahren 1:

- Vorlegen der oben beschriebenen hochkonzentrierten Kieselsäuredispersion, wobei das vorgelegte Volumen so bemessen ist, dass es die Gesamtmenge an benötigen Kieselsäuresinteraggregaten enthält.
- Langsames Zudosieren des Gesamtvolumens an disperser Phase unter ständigem Homogenisieren z.B. mittels eines schnellaufenden Rührers, schnellaufenden Dissolvers oder eines Rotor-Stator-Systems.

- Anschließend langsames Zudosieren des gewünschten Restvolumens an reiner homogener Phase gegebenenfalls unter ständigem Homogenisieren z.B. mittels eines schnellaufenden Rührers, schnellaufenden Dissolvers oder eines Rotor-Stator-Systems.

Verfahren 2:

- Vorlegen des Gesamtvolumens an disperser Phase.
- Langsames Zudosieren der oben beschriebenen hochkonzentrierten Kieselsäuredispersion unter ständigem Homogenisieren z.B. mittels eines schnellaufenden Rührers, schnellaufenden Dissolvers oder eines Rotor-Stator-Systems, wobei das zudosierte Volumen so bemessen ist, dass es die Gesamtmenge an benötigen Kieselsäuresinteraggregaten enthält.
- Anschließend langsames Zudosieren des gewünschten Restvolumens an reiner homogener Phase gegebenenfalls unter ständigem Homogenisieren z.B. mittels eines schnellaufenden Rührers, schnellaufenden Dissolvers oder eines Rotor-Stator-Systems

Die beschriebenen Verfahren können sowohl in kontinuierlicher als auch in diskontinuierlicher Form durchgeführt werden. Bevorzugt ist die kontinuierliche Form.

Die Temperatur der flüssigen Phase während des Emulgierprozesses liegt zwischen 0 °C und 80 °C, bevorzugt zwischen 10 °C und 50 °C, besonders bevorzugt zwischen 20 °C und 40 °C.

Der Emulgierprozess kann bei Normaldruck, also bei 900 bis 1100 hPa, bei erhöhtem Druck oder im Vakuum durchgeführt werden. Bevorzugt ist der Prozess bei Normaldruck.

Die erfindungsgemäßen Emulsionen können für alle Zwecke eingesetzt werden, für die bereits bisher Emulsionen verwendet werden. Insbesondere sind dies wasserbasierende Beschichtungs-, Kleb- und Dichtstoffe, enthaltend z.B. Organosiliciumverbindungen, wie Organo(poly)silane, Organo(poly)siloxane, Organo(poly)silazane und Organo (poly)silcarbane; Polyolefine, wie silylterminierte Polyisobutylene (z.B. erhältlich unter der Marke Epion von Kaneka Corp., Japan); gesättigte oder ungesättigte Polyester wie z.B. Polykondensate aus Phthalsäuren und 1,2-Propandiolen bzw. Poly-co-kondensate aus Phthalsäuren, 1,2-Propandiolen und Maleinsäuren gegebenenfalls gelöst in Reaktivverdünnern wie Styrolen; Polyurethane, Polyole, wie Hydroxy-haltige Polyester, Hydroxy-haltige Polyether, Methyldimethoxysilylpropyl terminierte Polypropylenglycole (z.B. erhältlich als "MS-Polymere" von der Fa. Kaneka Corp. Japan), Hydroxy-haltige Polyacrylate; Polyisocyanate, wie aliphatische und aromatische Polyisocyanate, Isocyanat terminierte Polyurethan-Präpolymere, hergestellt durch Umsetzung von Polyolen mit Polyisocyanaten im Überschuss, sowie deren silyterminierte Derivate (z.B. erhältlich unter der Bezeichnung DESMOSEAL® von der Bayer AG, Deutschland);Polyurethane oder deren Vorstufen wie z.B. Polyetherpolyole, Polyacrylatpolyole, Polyesterpolyole, multifunktionelle Isocyanate wie Hexandiisocyanat, Toluendiisocyanat, Diphenylmethandiisocyanat bzw, mit blockierenden Schutzgruppen versehene Isocyanate wie Hydroxylamine oder Malonesterderivate; (Poly)Epoxyverbindungen, wie Bisphenol A basierende Epoxide, monomere, oligomere und polymere Glycidoxy-Funktionen enthaltende Verbindungen, wie Diglycidylether basierend auf Bisphenol A, Epoxy-Novolac-Grundstoffe und Harze, Epoxyalkydharze, Epoxyacrylate, aliphatische Epoxide wie lineare Alkylenbisglycidylether und cycloaliphatische Glycidylether, wie 3,4-Epoxycyclohexyl-3,4-Epoxy-Cyclohexane-Carboxylate und aromatische Epoxide, wie Triglycidylether von p-Aminophenol und Triglycidylether von Methylendianilin; (Poly)Amine, wie cyclische und lineare Amine, wie Hexamethylendiamin, aromatische Amine, wie 4,4'-Methylen-bis(2,6-diethylanilin), Bis-(2-aminoalkyl)-Polyalkylenoxid, wie Bis-(2-aminopropyl)-Polypropylenglycol und Jeffamine, (Poly)Amidoamine, (Poly)Mercaptane, (Poly)Carbonsäure, (Poly)Carbonsäureanhydride; Acrylate und deren Ester, wie Glycidylacrylate, Alkylacrylate und deren Ester, Methacrylate und deren Ester, Polysulphide bildende Polymere und Polysulfide, wie Thioplaste (z.B. erhältlich unter der Marke Thiokol der Fa. Toray Thiokol Co. Ltd.).

Beispiele für Epoxyverbindungen sind Alkylenbisglycidylether, wie

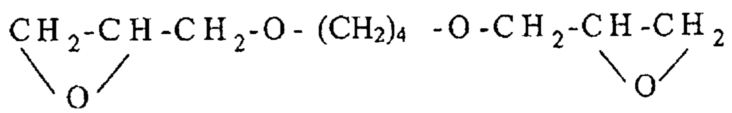

Bisphenol A basierende Diglycidylether, wie

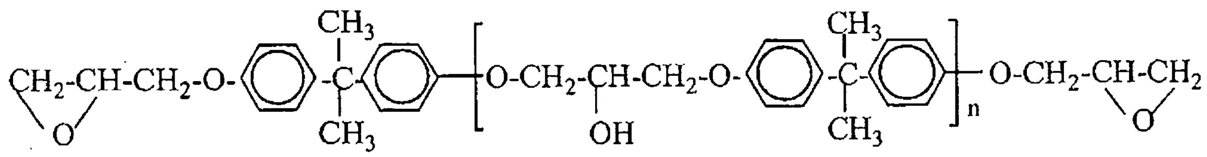

mit n bevorzugt von 0 bis 10, besonders bevorzugt 0 bis 5
Beispiele für Epoxy-Novolac-Harze sind solche der Formel

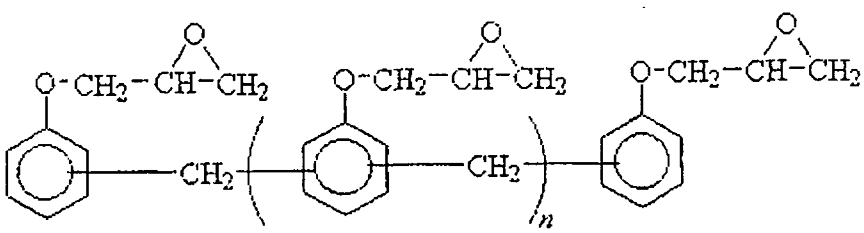

bifunktionelle Epoxyverbindungen, wie

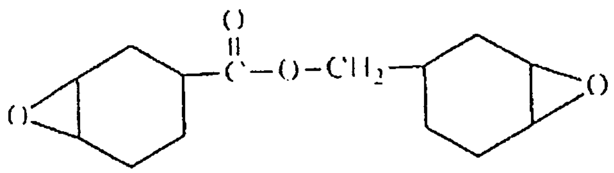

trifunktionelle Epoxyverbindungen, wie

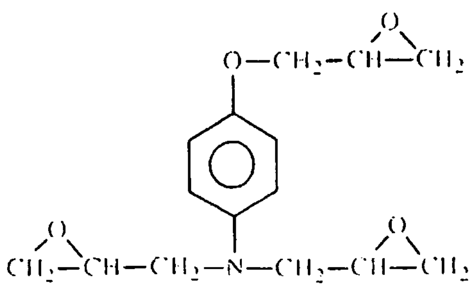

tetrafunktionelle Epoxyverbindungen, wie

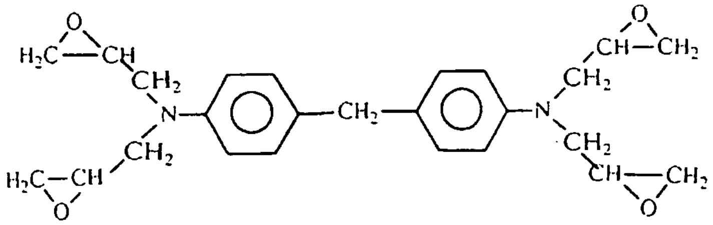

Ferner können die erfindungsgemäßen Emulsionen eingesetzt werden für kosmetische und pharmazeutische Anwendungen, Putz- und Reinigungsmittel oder Anwendungen zur Veränderung der Grenzflächeneigenschaften von festen und flüssigen Substraten, wie z.B. Hydrophobiermittel, Haftvermittlern, Trennmitteln, Papierbeschichtungen oder Schaumkontrollmitteln.
Ferner können die erfindungsgemäßen Emulsionen zur Herstellung von w/o/w- bzw. o/w/o-Mehrfachemulsionen verwendet werden, beispielsweise als Controll-Release-Systeme oder zur Segregation von Reaktivstoffen.
Die erfindungsgemäßen Emulsionen haben den Vorteil, dass sie weitgehend stabil gegen Separation, das heißt weitgehend stabil gegen Aufrahmen oder Sedimentation der dispersen Phase sind.
Die erfindungsgemäßen Emulsionen haben weiter den Vorteil, dass sie niedrige Scherviskositäten aufweisen und somit eine leichte Applikation ermöglichen.

**Beispiel 1:**

Herstellung von Feststoffpartikeln

100 g einer pyrogenen Kieselsäure mit einer spezifischen BET-Oberfläche, gemessen nach DIN 66131 und DIN 66132, von 200 $m^2/g$ (erhältlich bei Wacker-Chemie GmbH, D-München unter dem Namen Wacker HDK® N20) werden unter Rühren (mit 1000 UpM bei einem Rührflügeldurchmesser von 12,5 cm) fluidisiert, dann während 15 Minuten mit Stickstoffgas beaufschlagt und inertisiert, anschließend der Stickstoffstrom wieder abgestellt. Dann werden 2 g Dimethyldichlorsilan mit einer Zweistoffdüse als Aerosol in die fluidisierte Kieselsäure aufgesprüht, bei einer Temperatur von ca. 25°C und einem Umgebungsdruck von ca. 1013 hPa. Nach 30 Minuten weiterem Rühren wird anschließend die so behandelte Kieselsäure für 2 Stunden bei 300°C in einem Ofen von 100 l Rauminhalt getempert, unter leichtem Stickstoffstrom von 1000 1/h.
Erhalten wird eine weiße pulverförmige Kieselsäure mit folgenden Eigenschaften:

- Die Kieselsäure ist bedingt, aber nicht vollständig Wasser-benetzbar; dies zeigt sich dadurch, dass sich von der Kieselsäure nur 16 Gew.-% in Wasser mit einem Ultraturrax zu einer für einen Tag stabilen fließfähigen Masse einarbeiten lassen, unter den gleichen Bedingungen und zu gleicher Viskosität aber 24 Gew.-% der Ausgangskieselsäure (HDK® N20), die vollständig wasserbenetzbar ist.
- Weitere Eigenschaften der Kieselsäure sind in Tabelle 1 zusammengefaßt.

Tabelle 1:

| Eigenschaft | Kieselsäure B1 nach Beispiel 1 |
|---|---|
| BET-Oberfläche | 184 $m^2/g$ |
| Restgehalt an nicht silylierten Kieselsäure-Silanolgruppen | 80% |
| Kohlenstoffgehalt %C | 0,5 Gew.-% |
| Methanolzahl | 0 |
| Kontaktwinkel THETA Methode-1 gegen Wasser und Luft | 84° |
| Kontaktwinkel THETA Methode-2 gegen Wasser und Luft | von 80° |
| Oberflächenenergie GAMMA | 69 $mJ/m^2$ |
| Dispersionsanteil der Oberflächenenergie GAMMA-s-D | 65 $mJ/m^2$ |

- Spezifische BET-Oberfläche, gemessen nach DIN 66131 und DIN 66132
- Restgehalt an nicht silylierten Kieselsäure-Silanolgruppen, erhalten als Quotient (a) der Menge der Kieselsäuresilanolgruppen, der wie o.g. hergestellten Kieselsäure dividiert durch die Menge der Kieselsäuresilanolgruppen der unbehandelten Ausgangskieselsäure (Wacker HDK® N20); die Menge der Kieselsäuresilanolgruppen wird durch Säure-Base-Titration bestimmt (analog G.W. Sears Anal. Chem, 28 (12), (1950), 1981). Methode: Säure-Base-Titration der in Wasser/Methanol = 50:50 suspendierten Kieselsäure; Titration im Bereich oberhalb des pH-Bereichs des isoelektrischen Punktes und unterhalb des pH-Bereichs der Auflösung der Kieselsäure; unbehandelte Kieselsäure mit 100% SiOH (Kieselsäure-Oberflächensilanolgruppen): SiOHphil = 1.8 SiOH / $nm^2$; silylierte Kieselsäure: SiOH-silyl; Restgehalt an nicht silylierten KieselsäureSilanolgruppen: %SiOH = SiOH-silyl/SiOH-phil*100%
- Kohlenstoffgehalt %C bestimmt mittels Elementaranalyse auf Kohlenstoff; Verbrennen der Probe bei über 1000°C im $O_2$-Strom, Detektion und Quantifizierung des entstehenden $CO_2$ mit IR; Gerät LECO 244
- Methanolzahl, gemessen wie folgt: Test (Volumen% MeOH in Wasser) der Benetzbarkeit mit Wasser-Methanol Gemischen = Methanolzahl (MZ): Einschütteln eines gleichen Volumens der Kieselsäure mit gleichem Volumen an Wasser-Methanol Gemisch; Start mit 0% Methanol; bei Nicht-Benetzung schwimmt Kieselsäure auf: Es ist ein Gemisch mit um 5 Vol% höherem MeOH Gehalt zu verwenden; bei Benetzung sinkt Kieselsäure ein: Anteil MeOH (%) in Wasser gibt Methanolzahl (MZ)
- Kontaktwinkel THETA Methode-1 gegen Wasser, gemessen wie folgt: Der Kontaktwinkel der Partikel wird durch sorgfältiges Herstellen, mit üblichen Methoden, eines Presslings der Kieselsäure und anschließender Bestimmung des Kontaktwinkels gegen Wasser, hier ein aufliegender Wassertropfen aus bidestilliertem Wasser an Luft durch digitale Bildauswertung erhalten.
  Der Kontaktwinkel θ definiert das Verhältnis der Oberflächenspannungen und -energien γ von Flüssigkeiten (1) und Feststoffen (s) in einem Gasraum (g) wie folgt.

$$\cos(\theta) = (\gamma(sl) - \gamma(sg)) / \gamma(lg)$$

  Die Oberflächenenergie ($mJ/m^2$) eines Feststoffes ist dimensionsgleich mit der Oberflächenspannung einer Flüssigkeit (mN/m), da gilt [J] = [N*m].
- Kontaktwinkel THETA Methode-2 gegen Wasser, gemessen wie mittels Imbibitionsmethode unter Verwendung der Lucas-Washburn-Gleichung, beruhend auf dem Einsaugen von bekannten und definierten Flüssigkeit, mit bekannter Oberflächenspannung, in ein definiertes Haufwerk, wie hier ein schwach verdichteter Pressling aus der Kieselsäure mit offener Porosität größer 0,25 und Porenradius r. Die Aufsauggeschwindigkeit dh/dt, bzw. die Höhe der aufgesaugten Flüssigkeitssäule h, berechnet aus der Massenaufnahme m an Flüssigkeit durch das Partikelhaufwerk gegen die Zeit t, sowie die Viskosität der aufgesaugten Flüssigkeit η sowie die Oberflächenspannung γ der aufgesaugten Flüssigkeit lassen bei bekanntem Partikelradius r mittels der Gleichung nach Lucas-Washburn (Washburn, E.W., Phys. Rev. 17, 273 (1921) und R. Lucas Kolloid Z. 23, 15 (1918) den Wert Cosinus von θ ( cos (θ) ) und damit den Kontakt- oder Randwinkel θ der Flüssigkeit gegen die Partikeloberfläche ermitteln; folgend J. Schoelkopf et al, J. Colloid. Interf. Sci. 227, 119-131 (2000)
  Als Flüssigkeit mit bekannter Oberflächenspannung werden Methanol-Wasser-Mischungen der Mischungsverhältnisse (Volumen Methanol zu Volumen Wasser) 0:100, 5:95, 10:90, 15:85, 20:80, 25:75, 30:70, 35:65, 40:60, 45: 55, 50:50, 55:45, 60:40, 65:35, 70:30, 75:25, 80:20, 85:15, 90:10, 95:5, 100:0 verwendet.

$$dh/dt = r * \gamma * \cos(\theta) / (4 * \eta)$$

und

$$h^2 = r * \gamma * t * \cos(\theta) / (2 * \eta)$$

$t = A \cdot m^2$ Washburn-Gleichung

mit

t : Zeit

m : Masse der angesaugten Flüssigkeit

$$A = \frac{\eta}{\{C \cdot \rho^2 \cdot \gamma \cdot \cos \vartheta\}}$$

$\eta$ : Viskosität der Flüssigkeit
$\rho$ : Dichte der Flüssigkeit
$\gamma$ : Oberflächenspannung der Flüssigkeit
$\vartheta$ : Randwinkel Flüssigkeit-Pulver
C : Faktor, nur abhängig von den geometrischen Eigenschaften des Pulvers und Probenrohrs

Eine Illustration des Messverfahrens ist in Figur 5 zu finden.

- Die Oberflächenenergie GAMMA kann für Partikel als kritische Oberflächenenergie GAMMA-krit. mittels eines Zisman-Plots bestimmt werden, der wie in Figur 6 gegeben den jeweiligen Kontaktwinkel THETA der Kieselsäure gegen eine definierte Flüssigkeit, wie oben mit der Imbibitionsmethode bestimmt, gegen die Kontaktwinkel der jeweiligen Flüssigkeiten aufträgt.

- Für Partikel wie pyrogene Kieselsäure, die Agglomerate mit Schüttdichten $d_{SD}$ << 1 g/ml bildend, aber bestehend aus Primärpartikeln mit Materialdichten $d_{MD}$ > 1 g/ml, kann Einschütteln in Flüssigkeiten verschiedener Oberflächenspannung als Methode herangezogen werden: Bei Nichtbenetzung schwimmen die Partikel-Agglomerate auf; bei Benetzung wird die Luft in den Agglomeraten verdrängt, und die Partikel-Agglomerate sinken ein.
  Bei Verwendung verschiedener Flüssigkeiten mit verschiedener Oberflächenspannung kann exakt die Oberflächenspannung einer Flüssigkeit ermittelt werden, bei der die Partikel-Agglomerate einsinken; diese liefert die kritische Oberflächenenergie $\gamma_{crit}$ als Maß für die Oberflächenenergie $\gamma$ der Partikel.
  Die Methode kann auch dergestalt vereinfacht werden, dass die Oberflächenspannung von Wasser (72,5 mN/m) durch Zugabe von Methanol, Ethanol oder iso-Propanol verringert wird.
  Typischerweise kann Wasser vorgelegt werden, eine bestimmte Menge an Partikel-Agglomeraten auf die Wasseroberfläche aufgelegt (schwimmend) und dann der Alkohol, unter Rühren, zutitriert werden. Das Wasser zu Alkohol-Verhältnis bei Einsinken der Partikel-Agglomerate wird notiert und genau für dieses Verhältnis Wasser : Alkohol in einem getrennten Versuch mit Standardmethoden (Ringabreißmethode, Wilhelmy-Methode) die Oberflächenspannung bestimmt.
  Effektiver, und wie hier durchgeführt, werden definierte Mischungen von Wasser mit Methanol hergestellt, und dann werden die Oberflächenspannungen dieser Gemische bestimmt. In einem getrennten Experiment werden diese Wasser : Methanol Mischungen mit definierten Mengen an Partikel-Agglomeraten überschichtet (beispielsweise in einem Volumenverhältnis 1:1) und unter definierten Bedingungen geschüttelt (beispielsweise schwaches Schütteln mit der Hand oder mit einem Taumelmischer für ca. 1 Minute). Bestimmt wird das Wasser : Methanol-Gemisch, bei dem die Partikel-Agglomerate eben noch nicht einsinken und das Wasser : Methanol-Gemisch mit höherem Methanolgehalt, bei dem die Partikel-Agglomerate eben einsinken. Die Oberflächenspannung des letzteren Methanol : WasserGemisches liefert die kritische Oberflächenenergie $\gamma_{crit}$ als Maß für die Oberflächenenergie $\gamma$ der Partikel, wie in Tabelle 1 gegeben.

- Der Dispersions-Anteil der Oberflächenenergie gamma-s-D wird mit der Inversen Gaschromatographie und Alkanen als Sonden bestimmt, folgend "Inverse Gaschromatographie" - "Characterisation of Polymers and other Materials", 391 ACS Symposium Series, D R Lloyd, Th C Ward, H P Schreiber, Chapter 18, pp 248-261, ACS, Washington DC 1989, ISBN 0-8412-1610-X.

In einem 500 ml Edelstahlbecher werden mittels eines Dissolver mit Schlagzahnscheibe 16 g der oben beschriebenen teilhydrophoben Kieselsäure in 84 g vollentsalztem (VE)-Wasser vordispergiert. Die erhaltene hochviskose aber noch fließfähige Masse wird durch eine Ultraschallzelle mit einer Durchflußrate von 10 ml pro Minute und einer Amplitudenleistung von 300 Watt gepumpt. Die analytischen Daten der so erhaltenen Dispersion sind in Tabelle 2 zusammengefasst.

Tabelle 2:

| Eigenschaft | wäßrige Dispersion aus Beispiel 1 |
|---|---|
| Feststoffgehalt | 16,1% |
| pH-Wert | 5,3 |
| Mittlerer Durchmesser | 302 nm |
| Sinteraggregate | |
| Viskosität | 240 mPas |

- Feststoffgehalt der Dispersion bestimmt nach folgender Methode: 10 g wässrige Dispersion werden in einer Porzellanschale mit der gleichen Menge Ethanol versetzt und in einem $N_2$-gespülten Trockenschrank bei 150 °C zur Gewichtskonstanz eingedampft. Die Masse $m_S$ des trockenen Rückstandes ergibt den Feststoffgehalt gemäß Feststoffgehalt / % = $m_S$ * 100 / 10 g.
- pH-Wert gemessen mittels pH-Einstabmesskette
- Mittlerer Durchmesser der Sinteraggregate gemessen mittels Photokorrelationsspektroskopie nach folgender Methode: Von der zu messenden Dispersion werden 4 Proben mit einem Kieselsäuregehalt von 1 Gew.%, 0,75 Gew.%, 0,5 Gew.% und 0,25 Gew.% durch Einrühren der entsprechenden Menge Ausgangsdispersion mittels Magnetrührer in VE-Wasser hergestellt. Die Proben werden in einem PCS-Gerät Coulter N4 Plus der Fa. Coulter bei Detektionswinkeln von 30,1°, 62,6° und 90° vermessen. Der mittlere Durchmesser der Sinteraggregate ergibt sich aus einer Extrapolation der erhaltenen winkelabhängigen Meßwerte auf einen Kieselsäuregehalt von 0 Gew.% und anschließende Mittelung über die drei gemessenen Winkel.
- Viskosität der Dispersion bestimmt mit einem Rheometer MCR 600 der Fa. Haake mit Kegel-Platte-Sensorsystem (105 μm Meßspalt) bei 25 °C und einer Scherrate D = 10 $s^{-1}$.

78 g der oben beschriebenen Kieselsäuredispersion mit einem Feststoffgehalt von 16 Gew.% werden in einem 500 ml Edelstahlbecher vorgelegt. Unter Rühren bei 10000 rpm mit einem Ultraturrax und Wasserkühlung werden über einem Zeitraum von 15 min langsam 150 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60 °C steigen. Zu dieser nun hochviskosen standfesten Masse werden anschließend 86 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefaßt sind.

**Beispiel 2:**

78 g Kieselsäuredispersion gemäß Beispiel 1 werden in einem 500 ml Edelstahlbecher vorgelegt. Unter Rühren bei 10000 rpm mit einem Ultraturrax und Wasserkühlung werden über einem Zeitraum von 15 min langsam 150 g Nonansäureethylester zudosiert. Dabei sollte die Temperatur der Mischung nicht über 60 °C steigen. Zu dieser nun hochviskosen standfesten Masse werden anschließend 86 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefasst sind.

**Beispiel 3:**

150 g eines OH-terminierten Polydimethylsiloxans mit einer Viskosität von 1000 mPas (erhältlich unter dem Namen "OH 1000" bei Wacker-Chemie GmbH, D-München) werden in einem 500 ml Edelstahlbecher vorgelegt. Unter Rühren bei 10000 rpm mit einem Ultraturrax und Wasserkühlung werden über einem Zeitraum von 15 min langsam 78 g Kieselsäuredispersion gemäß Beispiel 1 zudosiert. Dabei sollte die Temperatur der Mischung nicht über 60 °C steigen. Zu dieser nun hochviskosen standfesten Masse werden anschließend 86 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefasst sind.

**Beispiel 4:**

78 g Kieselsäuredispersion gemäß Beispiel 1 werden in einem 500 ml Edelstahlbecher vorgelegt. Unter Rühren

bei 10000 rpm mit einem Ultraturrax und Wasserkühlung werden über einem Zeitraum von 15 min langsam 150 g einer Lösung von 125 g des Epoxyharzes Epikote 828 in 25 g Xylol zudosiert. Dabei sollte die Temperatur der Mischung nicht über 60 °C steigen. Zu dieser nun hochviskosen standfesten Masse werden anschließend 86 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefasst sind.

**Beispiel 5:**

In einem 500 ml Edelstahlbecher werden mittels eines Dissolver mit Schlagzahnscheibe 5 g einer teilhydrophoben Kieselsäure mit einem Hydrophobiergrad von 50% und einem Kohlenstoffgehalt von 1,1% (erhältlich unter dem Namen "Wacker HDK H20" bei Wacker-Chemie GmbH, D-München) in 85 g i-Dodecan eingerührt und anschließend 10 min bei 10000 rpm dispergiert. Zu dieser nun hochviskosen Dispersion werden unter Rühren bei 10000 rpm mit einem Ultraturrax und Wasserkühlung über einem Zeitraum von 15 min langsam 120 g VE-Wasser zudosiert. Dabei sollte die Temperatur der Mischung nicht über 60 °C steigen. Zu der resultierenden standfesten Masse werden anschließend 95 g i-Dodecan bei 1000 rpm langsam über einen Zeitraum von 15 min zugegeben. Dabei sollte die Temperatur der Mischung nicht über 60 °C steigen. Es resultiert eine dünnflüssige weiße W/O-Emulsion, deren analytische Daten in Tabelle 3 zusammengefasst sind.

**Beispiel 6**

94 g der Kieselsäuredispersion aus Beispiel 1 mit einem Feststoffgehalt von 16 Gew.% werden in einem 500 ml Edelstahlbecher vorgelegt. Unter Rühren bei 10000 rpm mit einem Ultraturrax und Wasserkühlung werden über einem Zeitraum von 15 min langsam 180 g eines Polydimethylsiloxans mit einer Viskosität von 100 mPas (erhältlich unter dem Namen "AK100" bei Wacker-Chemie GmbH, D-München) zudosiert. Dabei sollte die Temperatur der Mischung nicht über 60 °C steigen. Zu dieser nun hochviskosen standfesten Masse werden anschließend 41 g VE-Wasser ebenfalls bei 10000 rpm langsam über einen Zeitraum von 15 min zugegeben. Dabei sollte die Temperatur der Mischung nicht über 60 °C steigen. Es resultiert eine dünnflüssige weiße O/W-Emulsion, deren analytische Daten in Tabelle 3 zusammengefasst sind.

**Tabelle 3:**

| | Beispiel 1 | Beispiel 2 | Beispiel 3 | Beispiel 4 | Beispiel 5 | Beispiel 6 |
|---|---|---|---|---|---|---|
| Typ | O/W | O/W | O/W | O/W | W/O | O/W |
| $\eta_{rel}$ | 148 | 127 | 320 | 273 | 150 | 27 |
| [η] | 6,0 | 5,8 | 6,9 | 6,7 | 8,7 | 5,7 |
| $d_{50}$ / µm | 7,67 | 4,76 | 10,9 | 3,82 | 3,50 | 6,42 |
| $\Phi_w/\Phi_o$ | 50/50 | 50/50 | 50/50 | 50/50 | 40/60 | 40/60 |
| $V_{sep}$ / % | < 1 | < 1 | < 1 | < 1 | ca. 5 | ca. 8 |

## Patentansprüche

1. Emulsionen vom Typ Wasser-in-Öl (W/O) bzw. Öl-in-Wasser (O/W) enthaltend:

   - eine Ölphase (Phase A), enthaltend eine, gegebenenfalls mehrere, weitgehend wasserunlösliche Komponente (n),
   - eine Wasserphase (Phase B), die gegebenenfalls weitere wasserlösliche Komponenten wie Salze oder organische Verbindungen wie Alkohole, Carbonsäuren oder andere Verbindungen enthalten kann
   - an der Grenzfläche Öl-Wasser angeordnete pyrogene Kieselsäure, die in der Weise teilsilyliert ist, dass der Gehalt an nicht-silylierten Oberflächensilanolgruppen an der Siliciumdioxidoberfläche sich zwischen maximal 95% und minimal 5% des Ausgangs-Siliciumdioxids bewegt, gleichbedeutend mit 1,7 bis 0,1 SiOH-Gruppen pro $nm^2$ Kieselsäureoberfläche, der Dispersions-Anteil der Oberflächenenergie gamma-s-D 30 bis 80 $mJ/m^2$ beträgt, sowie die spezifische BET-Oberfläche einen Wert von 30 bis 500 $m^2$ /g aufweist,
   - und gegebenenfalls weitere Stoffe, wie Pigmente oder Konservierungsmittel,

wobei die Emulsionen eine mittlere Partikelgröße der dispersen Phase, d.h. einen mittleren Tropfendurchmesser, von 0,5 $\mu$m bis 500 $\mu$m aufweist, die Emulsionen niedrigviskos sind, wobei niedrigviskos bedeutet, dass die Emulsionen relative Viskositäten $\eta_r$ im Bereich von 1 bis $10^6$ aufweisen, wobei die relative Viskosität als der Quotient $\eta_{rel} = \eta/\eta_0$ der gemessenen Viskosität der Emulsion $\eta$, gemessen bei 25 °C und einer Scherrate D = 10 $s^{-1}$, dividiert durch die Viskosität der reinen homogenen Phase $\eta_0$ definiert ist
und die relative Viskosität $\eta_{rel}$ der Emulsion der Formel $\eta_{rel} = (1-\Phi/0{,}74)^{-([\eta]*0{,}74)}$ gehorcht, wobei $\Phi$ das Phasenvolumen der dispersen Phase und $[\eta]$ ein Formfaktor ist, der für die erfindungsgemäßen Emulsionen in einem Bereich von 2,5 bis 10 liegt.

**2.** Emulsionen nach Anspruch 1, **dadurch gekennzeichnet, dass** sie stabil gegen Separation der dispersen Phase sind, d.h. sie stabil gegen Aufrahmen bzw. Sedimentation der dispersen Phase sind, wobei stabil gegen Separation bedeutet, dass das Volumen der dispersionsverarmten Phase weniger als 10% des Gesamtvolumens des Gesamtvolumens beträgt.

**3.** Verfahren zur Herstellung der Emulsionen nach einem der Ansprüche 1 bis 2, **dadurch gekennzeichnet, dass** in einem ersten Schritt eine hochkonzentrierte feinteilige Dispersion der entsprechenden Kieselsäure in der Flüssigkeit, die in der Emulsion die homogene Phase bildet hergestellt wird und in einem zweiten Schritt eine hochviskose Präemulsion hergestellt wird, welche die Gesamtmenge der dispersen Phase und die im ersten Schritt hergestellte hochkonzentrierte feinteilige Dispersion der Kieselsäure in der Flüssigkeit, die in der erfindungsgemäßen Emulsion die homogene Phase bildet aufweist, wobei das eingesetzte Volumen der Dispersion so bemessen ist, dass die gesamte Menge der benötigen Kieselsäure enthalten ist, und in einem dritten Schritt die restliche homogenen Phase langsam eindosiert wird.

**4.** Verwendung der Emulsionen nach Anspruch 1 oder 2 als Beschichtungs-, Kleb- und Dichtstoffe, Emulsionen für kosmetische und pharmazeutische Anwendungen, Putz- und Reinigungsmittel oder Anwendungen zur Veränderung der Grenzflächeneigenschaften von festen und flüssigen Substraten, wie Hydrophobiermitteln, Haftvermittlern, Trennmitteln, Papierbeschichtungen oder Schaumkontrollmittel sowie zur Herstellung von W/O/W- bzw. O/W/O -Mehrfachemulsionen als Controll-Release-Systeme oder zur Segregation von inerten und reaktiven Stoffen.

**5.** Beschichtungs-, Kleb- und Dichtstoffe, die Emulsionen nach Anspruch 1 oder 2 enthalten.

**6.** Putz- und Reinigungsmittel, die Emulsionen nach Anspruch 1 oder 2 enthalten.

**7.** Hydrophobiermittel, Haftvermittler, Trennmittel, Papierbeschichtungen oder Schaumkontrollmittel, die Emulsionen nach Anspruch 1 oder 2 enthalten.

**8.** W/O/W- bzw. O/W/O-Mehrfachemulsionen, die Emulsionen nach Anspruch 1 oder 2 enthalten.

## Claims

**1.** Emulsions of the water-in-oil (W/O) or oil-in-water (O/W) type, containing:

- an oil phase (phase A), containing one substantially water-insoluble component or optionally a plurality of substantially water-insoluble components,
- a water phase (phase B) which may optionally contain further water-soluble components, such as salts or organic compounds, such as alcohols, carboxylic acids or other compounds,
- pyrogenic silica which is arranged at the oil-water interface and is partly silylated in a manner such that the content of non-silylated surface silanol groups on the silica surface is from not more than 95% to not less than 5% of the starting silica, equivalent to from 1.7 to 0.1 SiOH groups per $nm^2$ of silica surface, the dispersion fraction of the surface energy gamma-s-D is from 30 to 80 $mJ/m^2$ and the specific BET surface area has a value of from 30 to 500 $m^2/g$,
- and optionally further substances, such as pigments or preservatives,

the emulsions having a mean particle size of the disperse phase, i.e. a mean drop diameter, of from 0.5 $\mu$m to 500 $\mu$m, the emulsions having a low viscosity, low viscosity meaning that the emulsions have relative viscosities $\eta_r$ in the range of from 1 to $10^6$, the relative viscosity being defined as the quotient $\eta_{rel} = \eta/\eta_0$ of the measured viscosity of the emulsion $\eta$, measured at 25°C and a shear rate D = 10 $s^{-1}$, divided by the viscosity of the pure homogeneous phase $\eta_0$,

and the relative viscosity $\eta_{rel}$ of the emulsion obeys the formula $\eta_{rel} = (1-\Phi/0.74)^{-([\eta]\cdot 0.74)}$, $\Phi$ being the phase volume of the disperse phase and [η] being a form factor which is in a range of from 2.5 to 10 for the emulsions according to the invention.

**2.** Emulsions according to Claim 1, **characterized in that** they are stable to separation of the disperse phase, i.e. they are stable to creaming or sedimentation of the disperse phase, stable to separation meaning that the volume of the phase depleted in dispersion is less than 10% of the total volume.

**3.** Process for the preparation of the emulsions according to either of Claims 1 and 2, **characterized in that** a highly concentrated finely divided dispersion of the corresponding silica in the liquid which forms the homogeneous phase in the emulsion is prepared in a first step, and a highly viscous preemulsion which comprises the total amount of the disperse phase and the highly concentrated finely divided dispersion of the silica, prepared in the first step, in the liquid which forms the homogeneous phase in the emulsion according to the invention is prepared in a second step, the volume of dispersion used being such that the total amount of the silica required is present, and the remaining homogeneous phase being slowly metered in in a third step.

**4.** Use of the emulsions according to Claim 1 or 2 as coating materials, adhesives and sealants, emulsions for cosmetic and pharmaceutical applications, cleaning and cleansing agents or applications for changing the interfacial properties of solid and liquid substrates, such as water repellents, adhesion promoters, release agents, paper coatings or foam control agents and for the preparation of W/O/W or O/W/O multiple emulsions as control release systems or for the segregation of inert and reactive substances.

**5.** Coating materials, adhesives and sealants which contain emulsions according to Claim 1 or 2.

**6.** Cleaning and cleansing agents which contain emulsions according to Claim 1 or 2.

**7.** Water repellents, adhesion promoters, release agents, paper coatings or foam control agents which contain emulsions according to Claim 1 or 2.

**8.** W/O/W or O/W/O multiple emulsions which contain emulsions according to Claim 1 or 2.

## Revendications

**1.** Émulsions du type eau-dans-huile (E/H) ou huile-dans-eau (H/E), contenant :

- une phase huileuse (phase A), contenant un, éventuellement plusieurs, composant(s) dans une large mesure insoluble(s) dans l'eau,
- une phase aqueuse (phase B), qui peut éventuellement contenir d'autres composants hydrosolubles tels que des sels ou des composés organiques tels que des alcools, des acides carboxyliques ou d'autres composés,
- de la silice pyrogéné, disposé à l'interface huile-eau, qui est partiellement silylé, de sorte que la teneur en groupes silanol superficiels non silylés à la surface de la silice est comprise entre au maximum 95 % et au minimum 5 % de la silice de départ, ce qui est équivalent à 1,7 - 0,1 groupes SiOH par $nm^2$ de surface de la silice, la proportion de dispersion de l'énergie superficielle gamma-s-D vaut de 30 à 80 $mJ/m^2$ , et la surface spécifique BET a une valeur de 30 à 500 $m^2/g$,
- et éventuellement d'autres substances, telles que des pigments ou des conservateurs,

les émulsions ayant une taille moyenne de particule de la phase dispersée, c'est-à-dire un diamètre moyen de goutte, de 0,5 μm à 500 μm, les émulsions étant peu visqueuses, peu visqueuses signifiant que les émulsions présentent des viscosités relatives $\eta_r$ dans la plage de 1 à $10^6$, la viscosité relative étant définie comme le quotient $\eta_{rel} = \eta/\eta_0$ de la viscosité η mesurée de l'émulsion, mesurée à 25 °C et à une vitesse de cisaillement D = 10 $s^{-1}$, divisée par la viscosité $n_0$ de la phase homogène pure
et la viscosité relative $\eta_{rel}$ de l'émulsion obéit à la formule $\eta_{rel} = (1-\phi/0,74)^{-([\eta]*0,74)}$, $\phi$ étant le volume de phase de la phase dispersée et [η] étant un facteur de forme, qui pour les émulsions selon l'invention se situe dans une plage allant de 2,5 à 10.

**2.** Émulsions selon la revendication 1, **caractérisées en ce qu'**elles sont stables vis-à-vis de la séparation de la phase dispersée, c'est-à-dire qu'elles sont stables vis-à-vis du crémage ou de la sédimentation de la phase dispersée, stables vis-à-vis de la séparation signifiant que le volume de la phase appauvrie en dispersion est inférieur à 10 %

du volume total.

3. Procédé pour la préparation des émulsions selon l'une quelconque des revendications 1 à 2, **caractérisé en ce que** dans une première étape on prépare une dispersion finement divisée, fortement concentrée, de la silice correspondant dans le liquide qui constitue la phase homogène dans l'émulsion et dans une deuxième étape on prépare une pré-émulsion hautement visqueuse qui comporte la quantité totale de la phase dispersée et la dispersion finement divisée, fortement concentrée, préparée dans la première étape, de la silice dans le liquide qui constitue la phase homogène dans l'émulsion selon l'invention, le volume utilisé de la dispersion étant choisi de manière que soit contenue la quantité totale de la silice requis, et dans une troisième étape on introduit lentement par addition dosée la phase homogène restante.

4. Utilisation des émulsions selon la revendication 1 ou 2, comme matières de revêtement, adhésifs et matières d'étanchéité, émulsions pour des applications cosmétiques et pharmaceutiques, des compositions d'enduits et de nettoyage ou des applications pour la modification des propriétés interfaciales de substrats solides ou liquides, tels que des agents d'hydrophobie, des promoteurs d'adhérence, des agents de séparation, des sauces de couchage de papier ou des régulateurs de mousse ainsi que pour la préparation d'émulsions multiples E/H/E ou H/E/H en tant que systèmes à libération contrôlée (control release) ou pour la ségrégation de substances inertes et de substances réactives.

5. Matières de revêtement, adhésifs et matières d'étanchéité, qui contiennent des émulsions selon la revendication 1 ou 2.

6. Compositions d'enduits et de nettoyage, qui contiennent des émulsions selon la revendication 1 ou 2.

7. Agents d'hydrophobie, promoteurs d'adhérence, agents de séparation, sauces de couchage de papier ou régulateurs de mousse, qui contiennent des émulsions selon la revendication 1 ou 2.

8. Émulsions multiples E/H/E ou H/E/H, qui contiennent des émulsions selon la revendication 1 ou 2.

Fig. 1

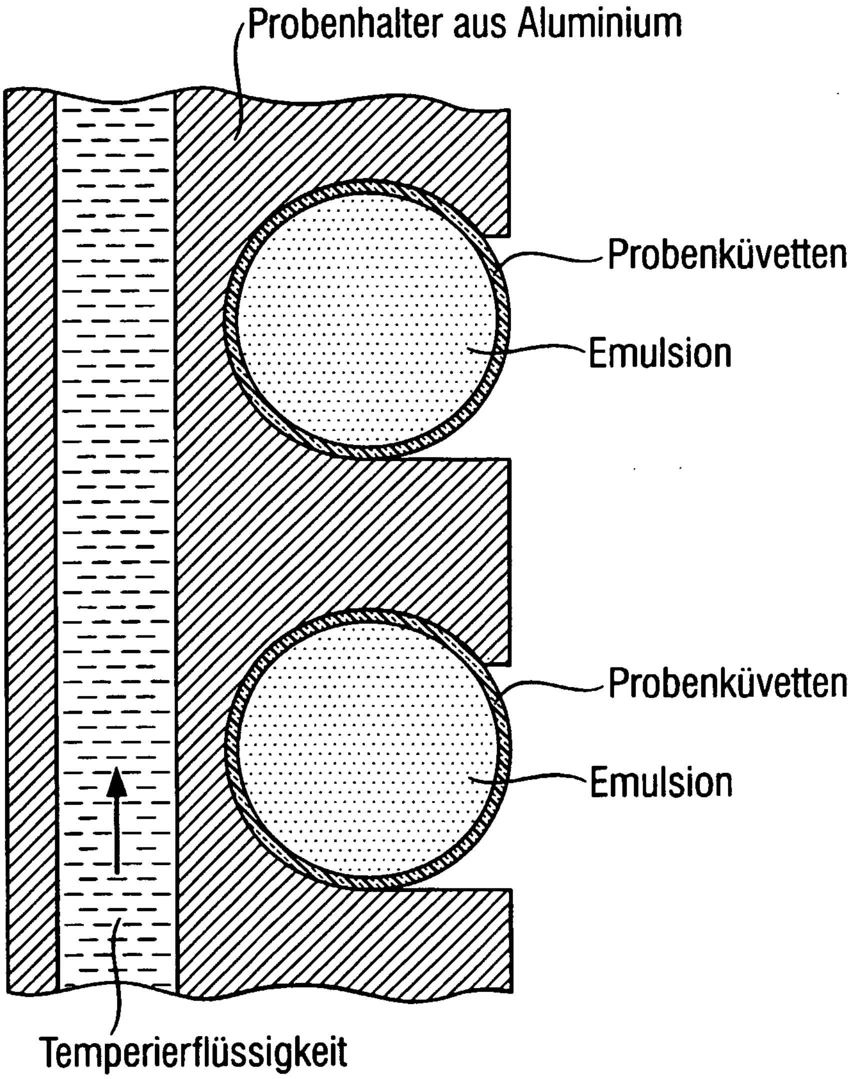
Probenhalter aus Aluminium
Probenküvetten
Emulsion
Probenküvetten
Emulsion
Temperierflüssigkeit

Fig. 2

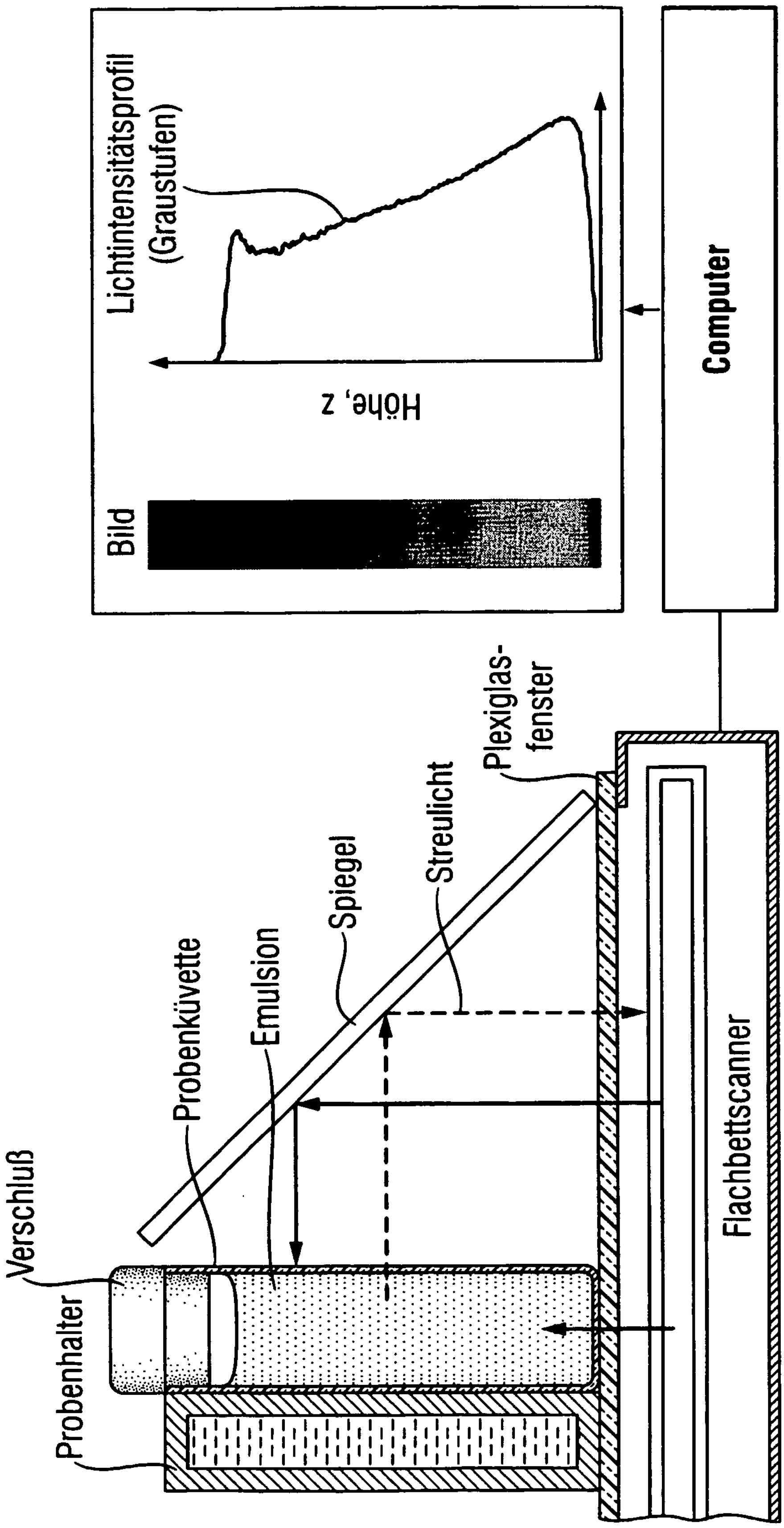
Probenhalter
Verschluß
Probenküvette
Emulsion
Spiegel
Streulicht
Plexiglas-
fenster
Flachbettscanner
Computer
Bild
Höhe, z
Lichtintensitätsprofil
(Graustufen)

Fig. 3

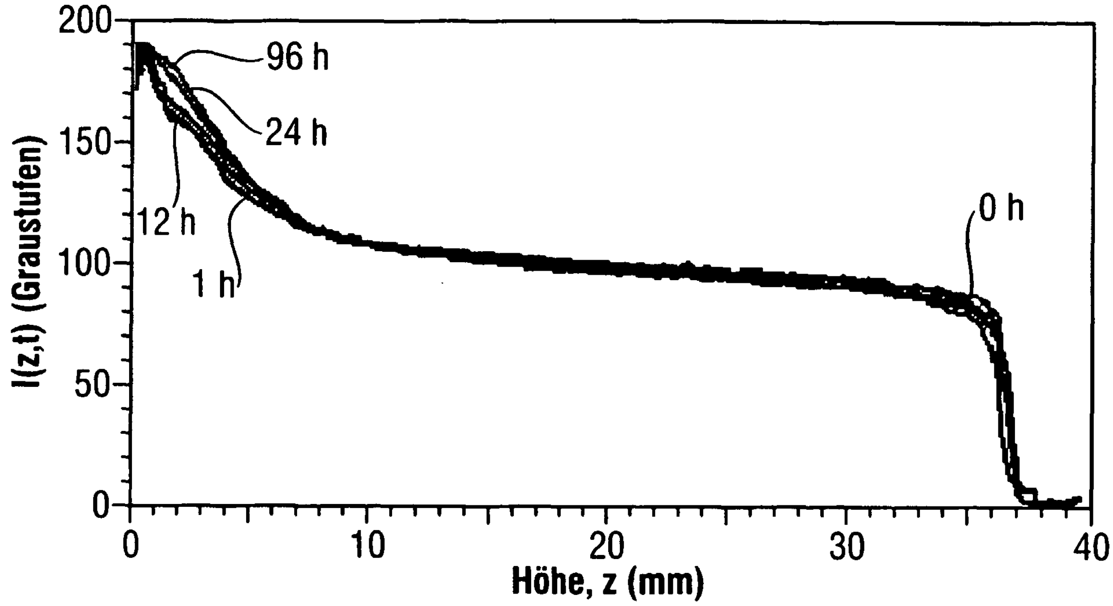
200
150
100
50
0
I(z,t) (Graustufen)
96 h
24 h
12 h
1 h
0 h
0
10
20
30
40
Höhe, z (mm)

Fig. 4

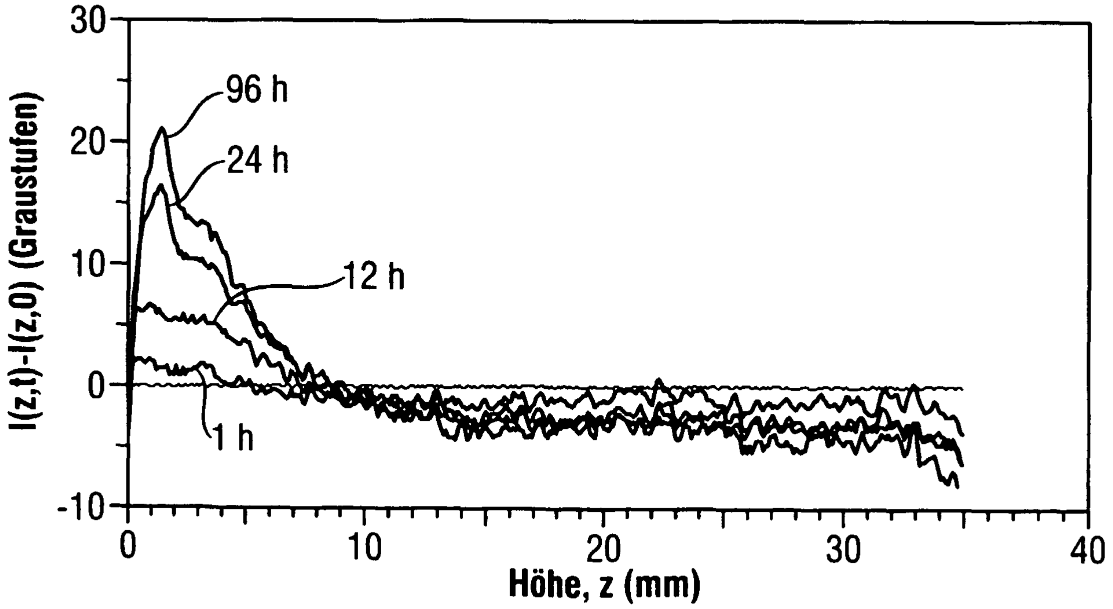
30
20
10
0
-10
I(z,t)-I(z,0) (Graustufen)
96 h
24 h
12 h
1 h
0
10
20
30
40
Höhe, z (mm)

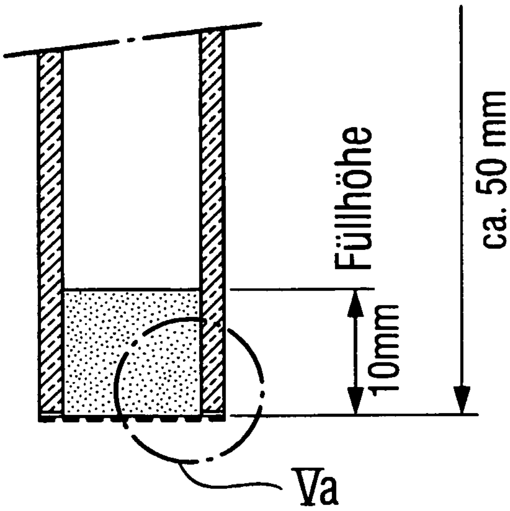


Fig. 5

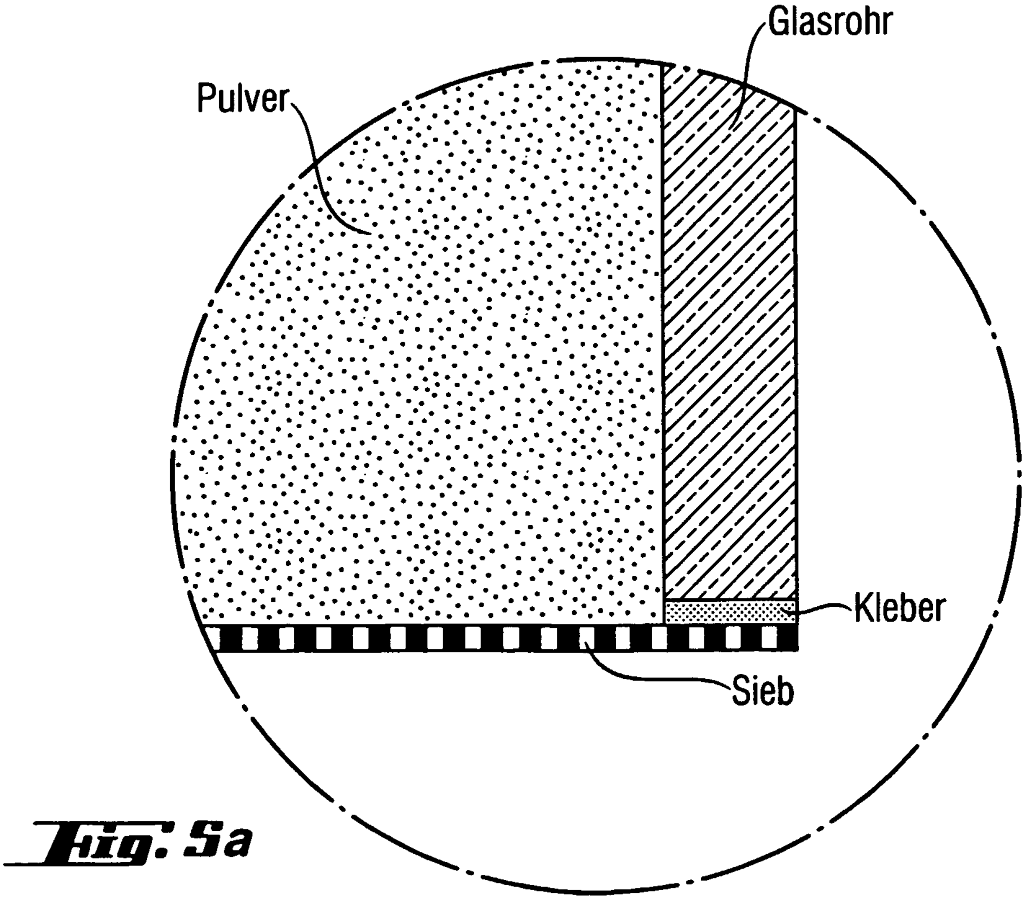


Fig. 5a

Fig. 6

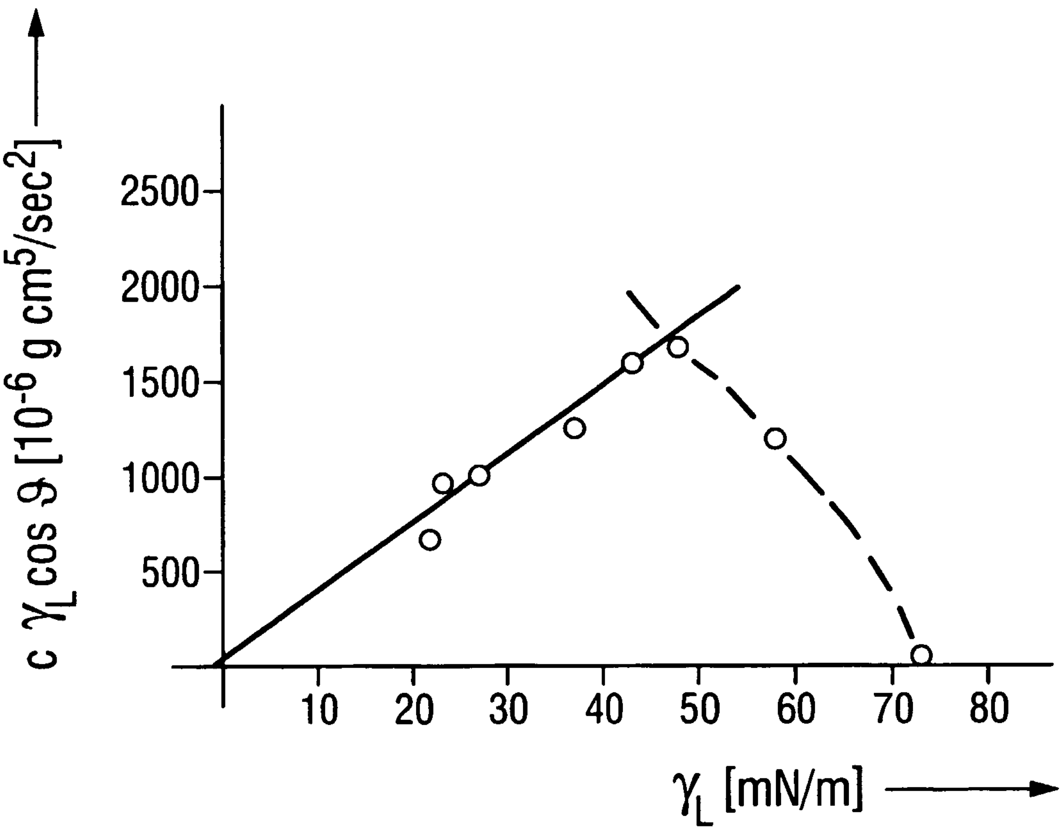
c γL cos ϑ [10-6 g cm5/sec2]
2500
2000
1500
1000
500
10
20
30
40
50
60
70
80
γL [mN/m]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 987008 A


### In der Beschreibung aufgeführte Nicht-Patentliteratur


- **R. de Rooij ; A. A. Potanin ; D. van den Ende ; J. Mellema.** *J. Chem. Phys.,* 1993, vol. 99, 9213
- **D R Lloyd ; Th C Ward ; H P Schreiber.** Inverse Gaschromatography''-''Characterisation of Polymers and other Materials. ACS Symposium Series, 1989, vol. 391, 248-261
- **G.W. Sears.** *Anal. Chem,* 1981, vol. 28 (12
- **Washburn, E.W.** *Phys. Rev.,* 1921, vol. 17, 273
- **R. Lucas.** *Kolloid Z.,* 1918, vol. 23, 15
- **J. Schoelkopf et al.** *J. Colloid. Interf. Sci.,* 2000, vol. 227, 119-131
- **D R Lloyd ; Th C Ward ; H P Schreiber.** Inverse Gaschromatographie'' - ''Characterisation of Polymers and other Materials. ACS Symposium Series, 1989, vol. 391, 248-261